# EUROPEAN PATENT APPLICATION

(11) **EP 4 617 667 A1**
(43) Date of publication of application: **17.09.2025**
(21) Application number: 24162990.6
(22) Date of filing: 12.03.2024
(51) Int. Cl.: G01N 33/68

(54) **EXOPHERS IN THE DIAGNOSIS, PROGNOSIS OR MONITORING OF DISEASE**

(71) Applicant: Universitätsklinikum Hamburg-Eppendorf, 20246 Hamburg (DE)
(72) Inventor: MEYER-SCHWESINGER, Catherine, 20246 Hamburg (DE); LAHME, Karen, 20246 Hamburg (DE); BREHLER, Michael, 20251 Hamburg (DE); BONN, Stefan, 20251 Hamburg (DE); ZIMMERMANN, Marina, 20251 Hamburg (DE)
(74) Representative: Stüven, Ralf

(57) **Abstract**

The invention relates to an in-vitro method for the diagnosis, prognosis or monitoring of a disease involving the detection, quantification, characterization or analysis of exophers in a sample taken from a subject. The invention can be used particularly advantageously in the diagnosis, prognosis or monitoring of chronic kidney disease, e.g. membranous nephropathy (MN).

## Description

The invention relates to an in-vitro method for the diagnosis, prognosis or monitoring of a disease involving proteostatic dysfunction. Further, the invention relates to isolated human exophers.

Exophers represent a novel type of extracellular vesicle (EV) recently discovered in *Caenorhabditis elegans* neurons and body wall muscles [46, 80]. Exophers extrude from juxtanuclear plasma membrane areas as long nanotubules which can reach up to 4 µm in diameter at the distal end [4, 5]. In *C*. *elegans,* exopher-formation depends on cellular stressors and mechanistically involves the delivery of cargo to aggresome-like organelles [5, 52, 57]. Exopher-like processes have morphologically been reported in mammalian systems and are thought to represent a protective strategy to remove proteotoxic protein aggregates and dysfunctional organelles such as mitochondria [4, 15, 22, 35, 51, 52, 69]. Phagocytosis of released exophers by neighboring cells is considered neuroprotective [46]. This export of proteotoxic protein aggregates may be conserved in flies and mammalian neurons [17, 55, 16].

Kidney diseases, in particular chronic kidney diseases (CKD) like membranous nephropathy are increasing, and are one of the most common causes for death worldwide (see, for example, [9, 30, 40]). Idiopathic membranous nephropathy (MN) is an autoimmune disease of kidney podocytes. Podocytes are essential cells of the filtration barrier to blood. In MN, circulating autoantibodies are directed against podocyte foot process proteins, such as the M-type phospholipase A2 receptor (PLA₂R1; [7]) in 80%, and thrombospondin type-1 domain-containing 7 A (THSD7A; [76]) in 5% of patients. Based on clinical correlative observations, the causes of autoimmunity are likely to be multiple in MN [8, 33, 34, 72, 87], as mirrored by the multitude of recently discovered potential MN autoantigens (reviewed in [34, 79, 68]). In the current concept of MN, the circulating autoantibodies cause the disease by complement-dependent as well as independent mechanisms [32, 38, 47, 57, 58, 65, 67, 77]. The ensuing autoantibody/antigen reaction leads to their pathognomonic glomerular deposition as immune complexes, finaly leading to glomerular filtration barrier alterations and nephrotic range proteinuria [25]. The underlying mechanisms and clinical significance of glomerular aggregate formation in MN are unknown. Currently, in MN, known diagnostic methods include kidney biopsies, detection of auto-antibody titers in serum, urine analysis (e.g. as regards the protein content), and other techniques, but not exophers.

It is an object of the invention to provide novel means for the diagnosis, prognosis or monitoring of a disease involving proteostatic dysfunction.

For solving the problem the invention provides, in a first aspect, a method for the diagnosis, prognosis or monitoring, in vitro, of a disease involving proteostatic dysfunction, comprising the step of
a) detecting the presence or quantity of exophers in a cell or tissue sample or in a body fluid sample from a subject; and/or
b) analyzing the contents of exophers in a cell or tissue sample or in a body fluid sample.

It has surprinsingly been shown that the detection and/or analysis of exophers, a recently discovered new type of vesicle, can advantageously be used for, e.g., diagnostic or prognostic purposes in a variety of diseases or conditons, for example, in the context of autoimmune diseases like chronic kidney disease, e.g. membranous nephropathy (MN). Without wanting to be bound to a particular theory, it is assumed that exophers play a significant role in the maintenance of cellular homeostasis, in particular in the removal of cell debris and potentially toxic protein aggregates [69]. Detection and/or analysis of exophers is thus advantegous in relation to any disease that involves proteostatic cell stress, e.g. taupathies or autoimmune diseases leading to or associated with protein aggregation. The invention thus provides a broadly applicable method for the diagnosis, prognosis or monitoring of a broad range of diseases, including autoimmune diseases and diseases that involve or are associated with proteotoxicity and, for example, the formation of aggresomes by cells. For example, in relation to chronic kidney disease, e.g., MN, exopher-formation and release is considered a protective pathomechanism to remove antigen/autoantibody aggregates from the podocyte. It is within the scope of the invention to detect and/or analyse exophers in a cell or tissue sample, or in a body fluid sample from a subject, for example a human. It is, however, preferred to detect and/or analyse exophers in a body fluid, for example in urine, cerebrospinal fluid, pleural fluid, peritoneal fluid, synovial fluid or pericardial fluid. In case of a urine sample, no invasive procedure is required. Tracking exopher-release to urine, for example, adds a non-invasive diagnostic tool with prognostic potential to clinical diagnostics and follow-up of kidney diseases, e.g. in MN patients.

The term "exopher" refers to large (average diameter approx. 400-600 nm, maximum size up to 8000 nm and even larger) lipid-bilayer membrane-enclosed extracellular vesicles (EVs) hanging at the end of a stalk (see, for example, [13, 54, 82]). Exophers arise frequently from the cell soma but may arise from cell processes. Exophers may have protein aggregates, cytosolic proteins, LC3 (MAP1LC3B), phosphatidylserine, Tau protein, Huntingtin, and whole organelles, e.g. damaged mitochondria, damaged proteasomes, disease-associated proteins such as complement, as well as aggresome proteins as a cargo or constituent [54, 36]. Exophers are Annexin V negative and Annexin A1 positive. They are jettisoned from the cell body within about 15-60 min in cell culture. Exophers can be distinguished from other extracellular vesicles by, for example, morphological criteria (extrusion from apical membranes especially from the juxtanuclear area as stalked large vesicles; expression of a characteristic set of EV markers [4, 5, 46]), dynamic criteria (enhanced release by proteotoxic stress, reuptake by cells, e.g., along the nephron [4, 15, 86], and molecular criteria (abundance of cell-specific and disease-associated proteins, presence of organelles [4, 6, 49]. Exophers differ from other extracellular vesicles, e.g. from migrasomes [45]. Migrasomes are large extracellular vesicles with small inner numerous vesicles that are formed at tips and intersections of rectraction fibres of migrating cells (see, e.g. [13, 54, 82]). They also differ in size and vesicular marker composition from EVs described by Hara et al. [31], which originate from tip vesiculation of podocyte microvilli.

The term "kidney-derived exophers" refers to exophers formed by kidney cells, for example, tubular cells or glomerular cells, e.g. podocytes. The term "tubule cell derived exopher" refers to exophers formed by kidney tubule cells, e.g. proximal tubule epithelial cells. The term "podocyte-derived exophers" refers to exophers formed by kidney podocytes. The terms "central nervous system derived exophers" and "myocardium-derived exophers" refers to exophers formed by cells of the central nervous system and the myocardium, respectively.

The term "extracellular vesicle" (EV) refers to lipid bilayer vesicles released by cells into the surrounding environment (see, e.g., [13, 18, 29, 44]). Examples of extracellular vesicles are microvesicles, exosomes, apoptic bodies, migrasomes and exophers.

The term "diagnosis, prognosis or monitoring" relates to the identification of a present disease, illness or physical condition, the prediction of the occurrence or the course of a disease, illness or physical condition, and to the observation of the course of a disease, illness or physical condition, for example, under a treatment.

The term "detecting the presence or quantity of exophers" refers to the qualitative and/or quantitative detection of exophers. The term "detecting the presence of exophers" includes the determination of the presence or absence of exophers. The term "detecting the quantity of exophers" includes the determination of the proportion of exophers in the totality of vesicles, for example, extracellular vesicles.

The term "analyzing the contents of exophers" refers to the ascertainment of the kind and/or amount of one or more of the constituents of exophers, e.g. the chemical compounds or molecules, molecule aggregates, organelles etc. it contains. The term "analyzing" encompasses the determination of the quantity or percentage of a component, e.g. a protein aggregate, in relation to other components and its biological activity state, e.g. enzymatic activity.

The term "autoimmune disease" refers to a disease, illness or physical condition resulting from the anomalous response of the adaptive immune system, i.e. of antibodies, against normal components of the own body. Autoimmune diseases typically involve the formation of antibodies against autoantigens (self-antigens), e.g. the body's own proteins.

The term "neurodegenerative disease" refers to a disease or disorder involving the progressive loss of structure or function of neurons. Neurodegenerative diseases include amyotrophic lateral sclerosis, multiple sclerosis, Parkinson's disease, Alzheimer's disease, Huntington's disease, multiple system atrophy, tauopathies, and prion diseases.

The term "kidney disease" refers to a disease or disorder involving a malfunction or dysfunction of the kidney, for example the tubules or the glomerulus. The term encompasses diseases or disorders due to damage and/or malfunctioning of, for example, tubular cells or glomerular cells, e.g. podocytes. The term "chronic kidney disease" (CKD), also "chronic kidney failure" refers to a disease resulting in a progressive loss of kidney function over a longer period (months or years) of time ([14, 21]). It is defined as the presence of kidney damage or an estimated glomerular filtration rate (eGFR) less than 60 ml/min per 1.73 square meters, persisting for 3 months or more [83].

The term "membranous nephropathy" (MN), also "membranous glomerulonephritis" (MGN) or "membranous glomerulopathy", refers to an autoimmune disease involving damage of the glomeruli of the kidneys [2, 61], in particular of the kidney podocytes. As used herein the term preferably relates to primary (idiopathic) MN. In idiopathic MN, autoantibodies attack podocyte foot process proteins, such as, for example, the M-type phospholipase A2 receptor (PLA₂R1; [7]) and thrombospondin type-1 domain-containing 7 A (THSD7A; [76]). Antigens and autoantibodies associated with membranous nephropathy are, for example, listed in [60].

The terms "disease involving proteostatic dysfunction" or "disease involving proteostatic cell stress" refer to a disease or disorder associated with a perturbation of proteostasis. The term "proteostasis" (protein homeostasis) refers to the ability of eukaryotic cells to maintain a stable proteome by acting on protein synthesis, quality control and/or degradation (53). The term includes, for example, inflammatory diseases, autoimmune diseases, neurodegenerative diseases, tauopathies or any other disease or disorder involving the formation of protein aggregates, within cells as aggresomes or outside of cells in form of deposits or plaques. The term "protein aggregate" refers to an accumulation and clumping of proteins where the aggregate or the aggregated protein(s) do not serve the original function of the aggregated proteins. The term not only encompasses protein aggregates of unfolded or misfolded proteins, but also aggregates of, for example, antigen/antibody complexes.

The term "tauopathy" refers to a disease or disorder characterized by the deposition of abnormal Tau protein, e.g. in cells of the central nervous system, in particular brain cells [20, 28, 88]. An example of a tauopathy is Alzheimer's disease. The term "tauopathy of the central nervous system" relates to a neurodegenerative disorder involving deposition of abnormal tau protein in central nervous system (CNS), i.e. the brain and spinal cord. The term "tauopathy of the myocardium" refers to a myocardial tauopathy". The term "tauopathy of the kidney" refers to a disorder involving deposition of abnormal tau protein in kidney cells, e.g. podocytes [84].

The term "14-3-3" relates to a family of ubiquitously expressed chaperone-like proteins [26]. In podocytes, 14-3-3 is a known synaptopodin interacting protein, playing a role in the downstream function of cyclosporin A [23].

The term "Hsc70" (also HSPA8, heat shock protein family A (Hsp70) member 8) relates to heat shock cognate 71 kDa protein, a constitutivley expressed chaperone protein [73]. Hsc70 is a member of the heat shock protein 70 (HSP70) family.

The term "Annexin A1" (ANXA1) refers to a protein gene of the annexin family of phospholipid-binding proteins [37].

The term "Annexin V" (ANXA5, see, e.g., NCBI Reference sequence NP_001145.1) refers to a protein of unknown function. Annexin V is a member of the annexin protein family, and binds phosphatidylserine with high affinity [12].

The term "α-Actinin-4" (ACTN4; see, e.g., NCBI Reference Sequences NP_004915.2, NP_001308962.1) refers to an actin-binding protein belonging to the spectrin gene superfamily [24].

The term "huIgG4" relates to human immunoglobulin G4 [59].

The disease involving proteostatic dysfunction can, for example, be an autoimmune disease, neurodegenerative disease or a tauopathy. In a preferred embodiment of the method of the invention the disease involving proteostatic dysfunction is a kidney disease. Further preferred, the disease involving proteostatic dysfunction is chronic kidney disease (CKD), further preferred membranous nephropathy (MN).

Preferably the sample is a body fluid sample, e.g., urine, cerebrospinal fluid, pleural fluid, peritoneal fluid, synovial fluid or pericardial fluid sample. Most preferred, the body fluid sample is a urine sample. Preferably, the sample is taken from a subject assumed to have, or is assumed to develop a disease involving proteostatic dysfunction, preferably a kidney disease, more preferably a glomerular disease, e.g. chronic kidney disease (CKD), more preferably membranous nephropathy (MN).

In a preferred embodiment of the method of the invention the exophers are kidney cell-derived exophers and the sample is a urine sample. Particularly preferred, the exophers are tubule cell derived exophers or podocyte-derived exophers, further preferred podocyte-derived exophers, and the sample is a urine sample.

In a particular preferred embodiment of the invention the method is a method for the diagnosis, prognosis or monitoring, in vitro, of membranous nephropathy, step a) is a step of detecting the presence or quantity of podocyte-derived exophers in a urine sample, and optional step b) is a step of analyzing the contents of the podocyte-derived exophers in the urine sample. In a preferred embodiment, the step a) of detecting the presence or quantity of podocyte-derived exophers in the urine sample comprises determining the proportion of podocyte exophers in the sample, normalized against the totality of vesicles in the sample.

In a preferred embodiment of the method according to the invention, the method comprises a step of enriching the exophers in the respective body fluid sample, e.g. urine sample. An enrichment of exophers in a body fluid sample can, for example, be accomplished by any method for enriching or isolating extracellular vesicles (EVs) from a body fluid (see, e.g., [42, 82]), for example, by differential centrifugation, size exclusion chromatography, filtration, or affinity-based methods, e.g. by immunoprecipitation ("pull-down") with the aid of matrix-associated antibodies, e.g. IgG, IgG4 or IgA coupled magnetic beads, or magnetic beads coupled with anti-14-3-3 and/or anti-Hsc70 and/or anti-Annexin A1 and/or other anti-aggresome proteins antibodies.

The exopher content can be analysed using any suitable known method. Examples are the biochemical analysis of the glomerular cell integrity such as protein-based methods (image stream, flow cytometry, immunoblots, immunoprecipitations, and proteomics), RNA-based methods (qPCR, transcriptomics), morphology-based methods (electron microscopy (EM), immunogold EM, immunofluorescence, high super-resolution microscopy, CryoEM, etc.). In a preferred embodiment of the method of the invention, the analysis of the contents of the exophers comprises the determination of a) the amount and characteristics of bound autoantibodies, b) the amount and characteristics of the protein contents, c) the amount and characteristics of the remaining molecules in the exopher and/or d) the functionality of organelles and/or protein complexes. An analysis of the contents of the exophers can be carried out using all suitable chemical, biochemical, physical and/or microbiological methods known to those skilled in the art.

Preferably, an analysis of the amount and characteristics of the protein content includes analysis of the amount, e.g. the absolute amount or proportions, and characteristics of autoantibodies, e.g. anti-PLA₂R1 and/or anti-THSD7A antibodies, and/or other autoantibodies associated with a particular disease, e.g. MN. It is to be noted that the analysis that will be carried out on the exopher content may depend on the particular disease to which the method of the invention is to be applied, for example, as regards the molecules, e.g. proteins, or structures that will be subjected to the analysis. The analysis may be qualitative and/or quantitative.

In a further preferred embodiment of the method of the invention step a) comprises the determination of the proportion of exophers in relation to the totality of extracellular vesicles, EVs, in the sample, preferably a body fluid sample, for example urine. This can, for example, be done using imaging flow cytometry ([56]) by labelling, e.g. via fluorophore labelling using membrane intercalating or membrane binding fluorophores, or fluorescently labeled antibodies, and distinguishing exophers from the remaining EVs by their Annexin-A1, 14-3-3, and/or Hsc70 positivity. huIgG4 and/or huIgG positivity may additionally be used as marker for exophers. Preferably, this embodiment of the method of the invention is carried out on a sample or a fraction of a sample in which extracellular vesicles have been enriched.

The method of the invention can also be carried out with a cell or tissue sample instead of a body fluid sample, e.g. with a biopsy sample. In a preferred embodiment of the method of the invention the sample is thus a cell or tissue sample, preferably a biopsy specimen. In this case, step a) preferably comprises the imaging of the deposition pattern of exophers in the sample. The imaging may comprise a suitable staining of the sample, e.g. a fluorescence staining. The analysis of the sample, including the identification of exophers, can, for example, be computer-assisted, e.g. by using a suitable algorithm for automatic detection and, for example, counting and measuring (signal strength and size) exophers in the sample. A computer-assisted analysis approach is, of course, also possible when using body liquid samples.

The subject from which the sample is taken, is preferably a mammal, more preferably a human. More preferably the subject is a human that has, is assumed to have, or is assumed to develop a disease involving proteostatic dysfunction, preferably a kidney disease, more preferably a glomerular disease, e.g. chronic kidney disease (CKD), more preferably membranous nephropathy (MN).

The method of the invention can be used in combination with or parallel to known diagnostic, prognostic or monitoring methods for the disease or disorder in question. For example, the method of the invention may be combined with the detection of autoantibodies in blood serum or urine. In MN, it is, for example, known to detect anti-PLA₂R1 antibodies in blood serum or in urine [85].

In a further aspect the invention also relates to a method for the enrichment or isolation of exophers from a body liquid sample of a subject, e.g. a human, preferably a urine sample. The method comprises the in-vitro binding of the exophers present in a body fluid sample to anti-14-3-3, and/or anti-huIgG4 and/or anti-Hsc70 antibodies immobilized to a solid support, e.g. affinity resin, magnetic beads or the like. The method may, for example, be carried out using immunoprecipitation. As an example, a body fluid sample, e.g. urine, or a sample, or fraction of a sample, of enriched or isolated extracellular vesicles, can be applied to a solid substrate, e.g. a resin or magnetic beads, having immobilized anti-14-3-3, and/or anti-huIgG4 and/or anti-Hsc70 antibodies. The exophers bind to the solid substrate and can subsequently be released from the substrate, and thus be isolated. Non-binding components of the fluid sample are removed.

In a further aspect the invention also relates to isolated human exophers. Preferably, the exophers are podocyte-derived exophers. The exophers can be isolated from a body fluid, e.g. cerebrospinal fluid, myocardial interstitium fluid, or urine. Exophers can be isolated as described above. Preferably, the exophers are isolated from a body fluid sample, or fraction of a body fluid sample, enriched in extracellular vesicles, the extracellular vesicles including the exophers. Enrichment of extracellular vesicles can be done by any suitable method (see above). Podocyte-derived exophers can, for example, be identified by the presence of podocyte-directed autoantibodies, e.g. anti-PLA₂R1 autoantibodies and/or anti-THSD7A autoantibodies, or podocyte-specific proteins. This includes complexes or aggregates of podocyte-directed autoantibodies and the respective antigen(s), e.g. aggregates of THSD7A and anti-THSD7A autoantibodies. Examples of podocyte-specific proteins include nephrin, podocin, α-Actinin-4, CD2-associated protein (CD2AP) and podoplanin (see, e.g., [41]). Further, podocyte-derived exophers can be identified by the protein 14-3-3, a chaperone-like protein [26]. MN-associated podocyte-derived exophers are huIgG4⁺/14-3-3⁺ EVs. Exophers of other origin, e.g. from other kidney cells, e.g. tubule cells, can be identified by markers, e.g. proteins, characteristic for these cells.

In a still further aspect, the invention relates to a computer implemented method for the automatic morphometric analysis of exophers in a tissue sample, the method comprising the following steps:
a) Preprocessing a digital grayscale image of a tissue section comprising a section through at least one glomerulus, the tissue section being stained for exophers, the preprocessing comprising:
   i. percentile normalizing the image, and
   ii. resampling the grayscale image resolution to a resolution of at least 0.049 µm per pixel,
b) median filtering the preprocessed image,
c) converting the median filtered image to a binary image,
d) separating signal clusters in the binary image from each other using a watershed transformation,
e) carrying out a Difference of Gaussian blob detection on the image generated in step d)
f) identifying detected blobs as exopher candidates,
g) determining the number, size, image intensity and/or centroids of exopher candidates identified in step f.

The algorithm can be implemented in any suitable programming language, (for example, in Python (e.g. Python 3). Stained images, for example, PLA₂R1-stained images, comprising at least one glomerulus section are used. Preferably, images in the CZI (Carl Zeiss Image) file format, that have been converted to grayscale images, are used as input.

The term "stained for exophers" means that the tissue section is stained in a manner such that the exophers or a component thereof are stained. A section can, for example, be exopher-stained by using a labelled anti-PLA₂R1 antibody, or another labelled antibody against a molecule that is associated with exophers, preferably against a molecule that is characteristic of exophers, for example 14-3-3 or Annexin A1.

The term "resampling the grayscale image resolution to a resolution of at least 0.049 µm per pixel" means that the resolution of the grayscale image is resampled to have a resolution such that a pixel covers at least 0.049 µm² of the tissue section.

The term "blob" refers to bright on dark or dark on bright regions in an image.

In a preferred embodiment of the computer implemented method for the automatic morphometric analysis of exophers, the method comprises, before step a), a step of creating a digital grayscale image of an original digital colour image. In this embodiment, the method also encompasses the step of generating the grayscale image used as input for the algorithm.

Step c) of converting the median filtered image to a binary image is preferably carried out using Multi-Otsu thresholding [43].

In a further preferred embodiment, the method comprises, after step c) of converting the median filtered image to a binary image, the step of
ci) removing artifacts from the binary image by
i. removing small objects having a size smaller than 40 pixels, and
ii. performing a morphological close operation with a size of 60.

In a still further preferred embodiment, the method comprises, after step c1 of removing artifacts from the binary image,
c₂) extracting a glomerular mask by calculating the convex hull around the binary signal of the section through the at least one glomerulus.

For a subsequent analysis, for example, the calculation of exopher density, the detected exopher candidates's number, size, image intensity and centroids (geometric center of each exopher) are saved.

Based on the detected centroids, the blob density is calculated for each blob. Blob density is a parameter defined as the sum of the number of neighboring blobs (excluding the current blob) within a certain radius (e.g. 50 pixels) around each blob divided by the total number of blobs in the image associated with a single glomerulus. In case a glomerular mask has been extracted, the total number of blobs in the complete masked area of the image is used. The division normalizes the density to a range from 0 (all blobs have 0 neighbors) to the total number of blobs in the (masked area of the) image (all blobs have all blobs as neighbors). The PLA₂R1 aggregated density per patient is calculated as the median of all PLA₂R1 densities of all images of that patient. Since each blob is considered an exopher candidate, the blob density is equivalent to the exopher candidate density.

In a further preferred embodiment of the computer implemented method for the automatic morphometric analysis of exophers, the method thus comprises, after step g), the step of h) calculating the blob density for each detected blob, the blob density being defined as the sum of the number of neighboring blobs around a certain blob within a certain radius around each blob divided by the total number of blobs associated with one glomerulus in the image.

In an embodiment of the method, in which a glomerular mask is extracted in a previous step, a division by the total number of blobs the total number of blobs in the complete masked area of the image is carried out in step h).

The invention is further illustrated using the attached figures and examples.

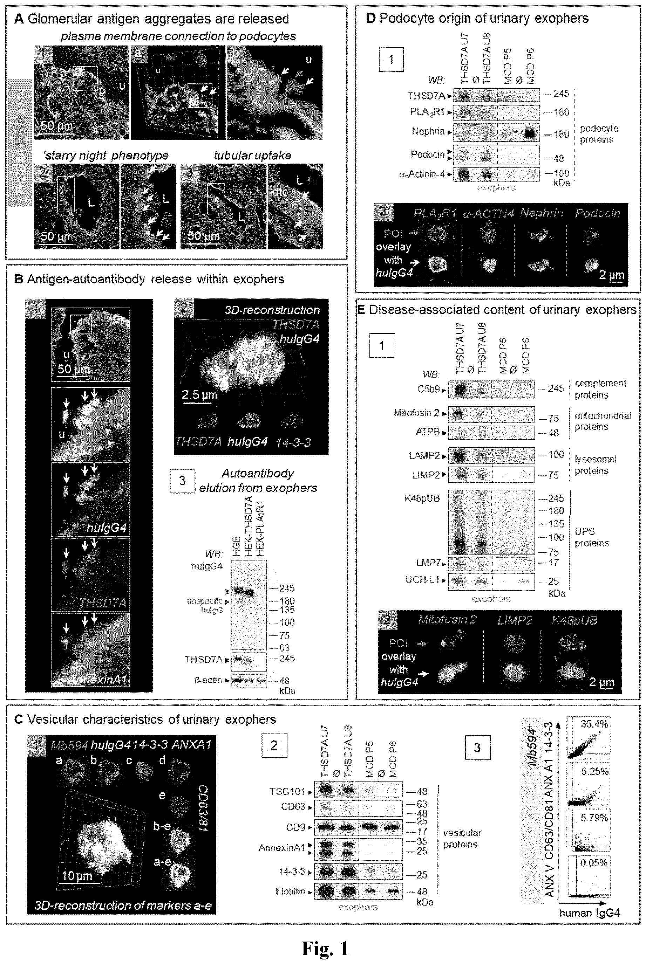

Figure 1: Glomerular urinary space aggregates are exophers in a THSD7A⁺-MN patient. The diagnostic biopsy and urines (U1-U8) collected from THSD7A⁺-MN patient P1 were evaluated for the presence of exophers. A) High-resolution confocal micrographs to THSD7A (white) and wheat germ agglutinin (dark gray, WGA, binds to N-acetyl-D-glucosamine and sialic acid of the glycocalyx and demarcates plasma membrane), DNA (light gray). *Panel 1:* Glomerular THSD7A aggregates localize to the subepithelial space (white arrowheads) and to the urinary (u) space (white arrows). Urinary space THSD7A aggregates exhibit a WGA⁺ membrane connection (gray arrow) to podocytes (p), *Panel 2:* THSD7A⁺ aggregates (white arrows) are found within the tubular lumen (L) or *panel 3* taken up by tubular cells, dtc = distal tubular cell. B) *Panel 1:* Confocal analyses demonstrate that THSD7A (gray) colocalizes with huIgG4 (white) in urinary space aggregates (white arrows). White arrowheads highlight huIgG4 in the subepithelial space. *Panel 2:* 3D-reconstructed confocal z-stack of a urinary exopher (from U1) stained for THSD7A (dark gray), huIgG4 (white) and the vesicle marker 14-3-3 (light gray). Lower row exhibits individual channels in one plane. *Panel 3:* Antibodies eluted from U2 exophers exhibit specific reactivity to THSD7A in human glomerular extract (HGE) and in HEK cells transduced with human THSD7A but not with human PLA₂R1. C) Urinary exophers express vesicle markers. *Panel 1:* 3D-reconstructed confocal z-stack of an exopher from U2 demonstrates expression of the vesicle markers 14-3-3 (light gray), Annexin A1 (light gray), CD63/CD81 (light gray). MemBrite594 (dark gray) stains the plasma membrane, huIgG4 (white) the bound autoantibody. *a-e:* individual channels in one plane. *Panel 2:* Immunoblot to vesicle markers in enriched exophers isolated from U7 and U8 in comparison to two nephrotic minimal change disease (MCD) patients P5 and P6. *Panel 3:* Image stream quantification of vesicle marker distribution on MemBrite594⁺huIgG4⁺ exophers in U1 discerns 14-3-3 as an abundant marker. Note the lack of Annexin V signal (marker for apoptotic bodies). D) Urinary exophers contain podocyte proteins. *Panel 1:* Immunoblot detection of podocyte-specific proteins in enriched exophers from U7 and U8 in comparison to the two nephrotic MCD patients. *Panel 2:* Confocal analyses demonstrate expression of podocyte-specific proteins (protein of interest (POI) in gray) within huIgG4⁺ (white) exophers of U2. E) Urinary exophers contain disease associated proteins. *Panel 1:* Immunoblot detection of disease associated proteins belonging to the complement cascade (C5b9, membrane attack complex), mitochondria (membrane proteins Mitofusin 2 and ATP synthase ATPB), lysosomes (membrane proteins LAMP2 and LIMP2), and the ubiquitin proteasome system (UPS: K48-polyubiquitinated proteins, the proteolytic β-subunit LMP7, the deubiquitinating enzyme UCH-L1) in enriched exophers isolated from U7 and U8 in comparison to the two nephrotic MCD patients. *Panel 2:* Confocal analyses demonstrate expression of Mitofusin 2, LIMP2, or K48pUB (all proteins of interest (POI) in gray) within huIgG4⁺ (white) exophers of U2.

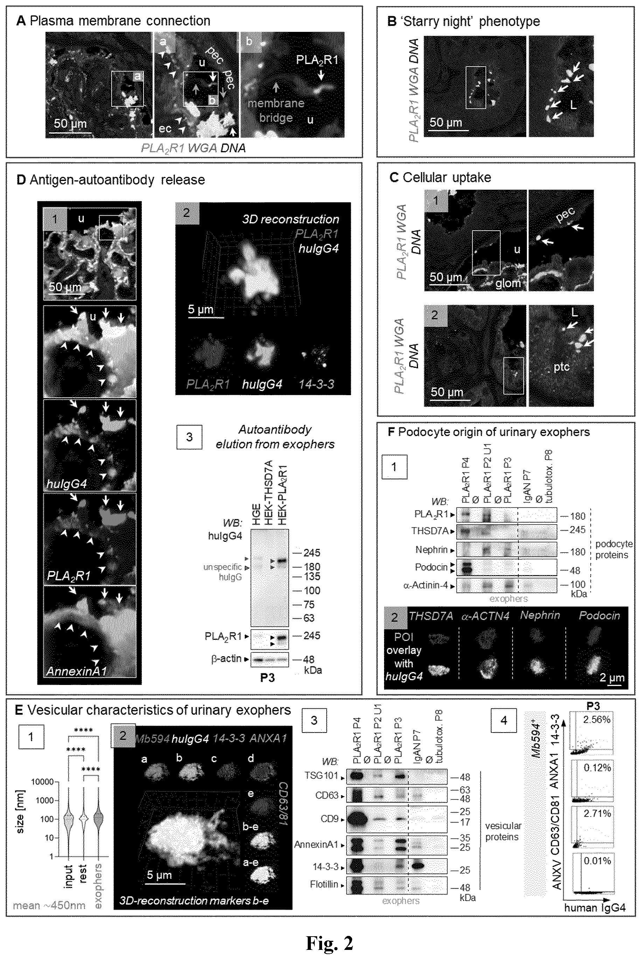

Figure 2. Exophers are found in PLA₂R1⁺-MN patients P2-P4. Diagnostic samples of P2, P3, and P4 PLA₂R1⁺-MN patients were analyzed for the presence of exophers. If not indicated otherwise, analyses from P2 urine U1 are shown. A) High-resolution confocal micrographs to PLA₂R1 (white) and wheat germ agglutinin (gray, WGA, binds to N-acetyl-D-glucosamine and sialic acid of the glycocalyx and demarcates plasma membrane), DNA (black). Glomerular PLA₂R1 aggregates localize to the subepithelial space (white arrowheads) and to the urinary (u) space (white arrows). Urinary space PLA₂R1 aggregates exhibit a WGA⁺ membrane connection (gray arrow) to podocytes. B, C) High-resolution confocal micrographs to PLA₂R1 (light gray) and WGA (dark gray), DNA (black) demonstrates PLA₂R1⁺ aggregates (white arrows) B) within the tubular lumen (L) and C) taken up by *panel 1*: parietal epithelial cells (pec) and *panel 2:* proximal tubular cells (ptc). D) *Panel 1:* Confocal analyses demonstrate PLA₂R1 (gray) colocalization with huIgG4 (light gray) in urinary space aggregates (white arrows). White arrowheads highlight huIgG4 and PLA₂R1 in the subepithelial space. *Panel 2:* 3D-reconstructed confocal z-stack of an exopher enriched from the patient urine demonstrates the high abundance of PLA₂R1 (dark gray) and huIgG4 (white) complexes. Lower row exhibits individual channels in one plane, vesicle marker 143-3 (light gray). *Panel 3:* Antibodies eluted from P3 exophers exhibit specific reactivity to PLA₂R1 in human glomerular extract (HGE) and in HEK cells transduced with human PLA₂R1 but not with human THSD7A. Reprobes to PLA₂R1 and β-actin control for molecular weight height and loading. E) Vesicular characteristics of PLA₂R1⁺-MN patient P2 exophers in U1. *Panel 1:* Nanoparticle tracking analyses of vesicular size distribution in total (input) urinary EVs, the exopher-depleted rest fraction and the exopher-enriched fraction. Violin plots indicate median, 25% and 75% percentile. Mean size of PLA₂R1⁺-MN exophers is ~450 nm. *Panel 2:* 3D-reconstructed confocal z-stack of an exopher demonstrates expression of the vesicle markers 14-3-3 (light gray), Annexin A1 (light gray), CD63/CD81 (light gray). MemBrite594 (dark gray) stains the plasma membrane, huIgG4 (white) the bound autoantibody. *a-e:* individual channels in one plane. *Panel 3:* Immunoblot to vesicle markers in enriched exophers isolated from the diagnostic urines of the 3 PLA₂R1⁺-MN patients in comparison to the nephrotic IgAN patient P7 and the tubulo-toxic kidney injury patient P8. *Panel 4:* Image stream analysis of vesicle marker distribution on MemBrite594⁺/huIgG4⁺ exophers discerns 14-3-3 as an abundant marker in PLA₂R1⁺-MN exophers collected from the urine of P3. Note the lack of Annexin V signal (marker for apoptotic bodies). F) Urinary exophers contain podocyte proteins. *Panel 1:* Immunoblot detection of podocyte-specific proteins in enriched exophers isolated from diagnostic urines of the PLA₂R1⁺-MN patients in comparison to the two nephrotic patients diagnosed with IgAN (P7) or tubulo-toxic kidney injury (P8). *Panel 2:* Confocal analyses demonstrate expression of podocyte-specific proteins (protein of interest (POI) in gray) within huIgG4⁺ (white) exophers of P2.

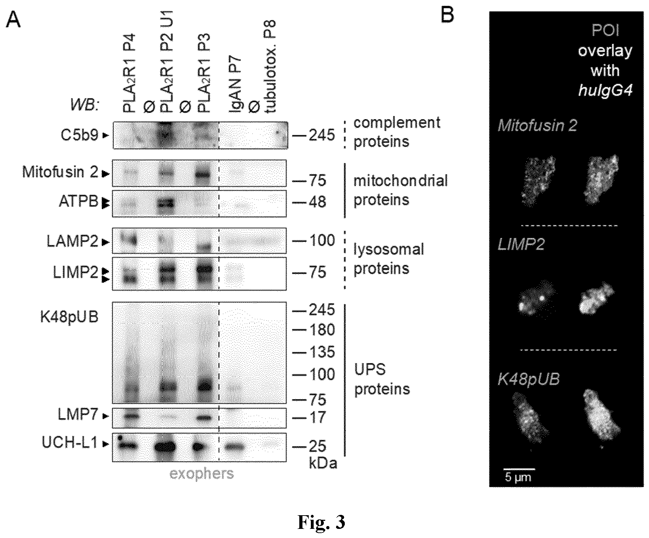

Figure 3. Urinary exophers enriched from the diagnostic urines of PLA₂R1⁺-MN patients P2-P4 contain disease-associated proteins. Urinary exophers enriched from diagnostic urine samples of P2, P3, and P4 PLA₂R1⁺-MN patients were analyzed for the presence disease-associated proteins. A) Immunoblot detection of disease associated proteins belonging to the complement cascade (C5b9, membrane attack complex), mitochondria (membrane proteins Mitofusin 2 and ATP synthase ATPB), lysosomes (membrane proteins LAMP2 and LIMP2), and the ubiquitin proteasome system (UPS: K48-polyubiquitinated proteins, the proteolytic β-subunit LMP7, the deubiquitinating enzyme UCH-L1) in enriched exophers from the diagnostic urines of the 3 PLA₂R1⁺-MN patients in comparison to the nephrotic IgAN patient P7 and the tubulo-toxic kidney injury patient P8. B) Confocal analyses demonstrate expression of Mitofusin 2, LIMP2, or K48pUB (all proteins of interest (POI) in gray) within urinary huIgG4⁺ (white) exophers of U1 of the PLA₂R1⁺-MN patient P2.

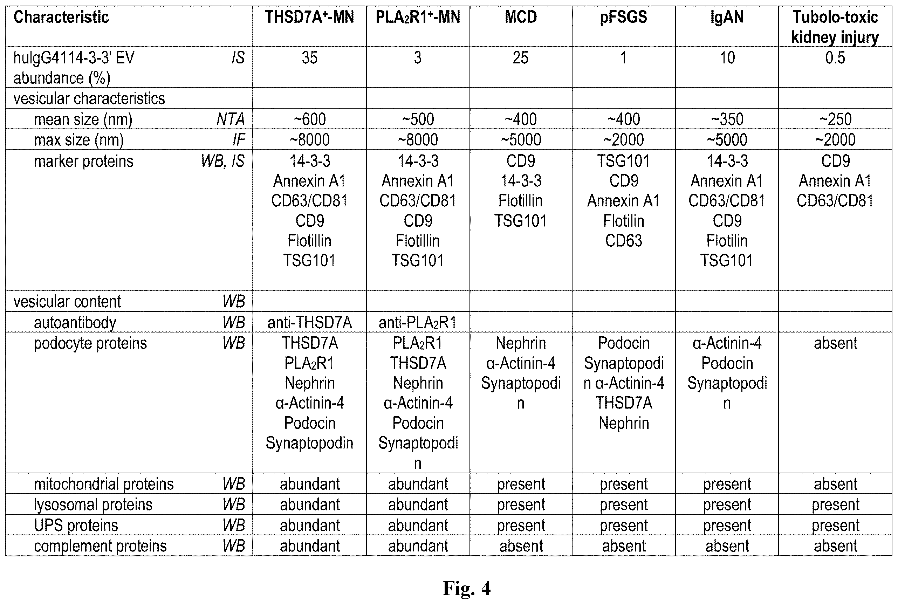

Figure 4. HuIgG⁺-EV characteristics in the diagnostic urines (U1) of nephrotic patients. Abbreviation: IS= image stream; NTA = nanoparticle tracking analyses; IF = immunofluorescence; WB = immunoblot; = approximately; MCD = minimal change disease; pFSGS = primary focal segmental glomerulosclerosis; IgAN = IgA nephropathy.

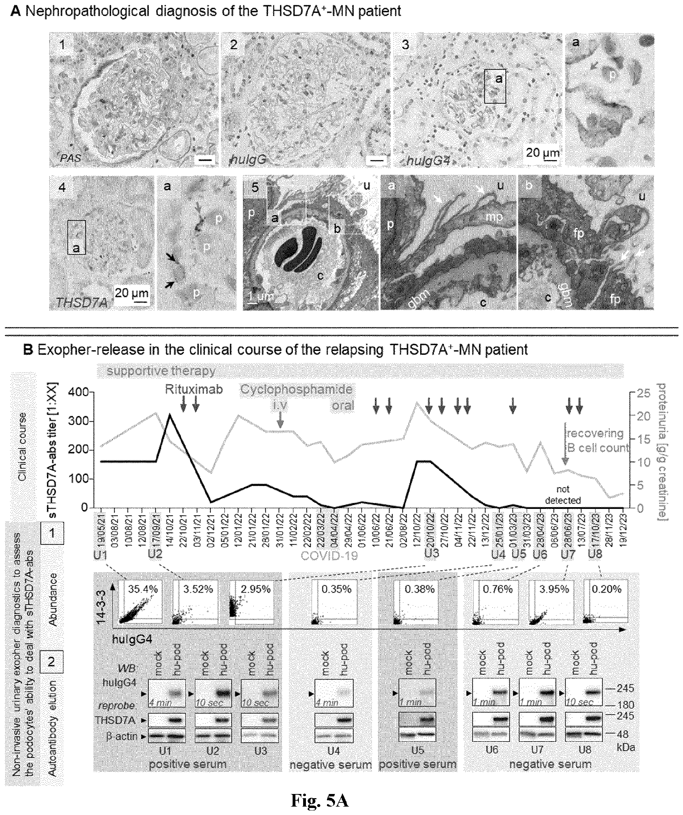

Figure 5A. Non-invasive exopher monitoring in relapsing THSD7A⁺-MN. A) Micrographs from the nephropathological diagnosis (19/05/2021) of the patient P1 encompassing light microscopy with *panel 1:* PAS staining for general morphology; immunohistochemistry to *panel 2:* huIgG, *panel 3:* huIgG4, and *panel 4:* THSD7A. Gray arrows highlight aggregates within the urinary space, black arrows aggregates within parietal epithelial cells. *Panel 5:* Transmission EM; white arrows highlight membrane extensions from major (*panel 5a*) and foot processes (*panel 5b*), p = podocyte, gbm = glomerular basement membrane, fp = foot process, mp = major process, u = urinary space, c = capillary space. B) Dynamics and characteristics of urinary exopher release over 2 ½ years of relapsing nephrotic syndrome. *Graph* depicts on the left y-axis the serum THSD7A autoantibody titer (black curve) and on the right y-axis urinary protein to creatinine ratio (gray curve). The main clinical interventions, as well as the COVID-19 infection are indicated. *Panel 1:* Image stream plots demonstrate the relative content of huIgG4⁺ exophers in the urine in % exophers from 10⁹ total EVs. *Panel 2:* Reactivity of the huIgG4 eluted from 10⁹ exophers to THSD7A of the 8 urines collected from this patient; hu-pod = human podocytes with human THSD7A overexpression, mock = human podocytes without THSD7A overexpression. Membranes were reprobed for THSD7A and β-actin to control for THSD7A expression/height and loading. The exposure times for the huIgG4 signal are indicated. Note the clear detection of THSD7A autoantibodies bound to released exophers in all urines, albeit a concomitant negative sTHSD7A-ab titer.

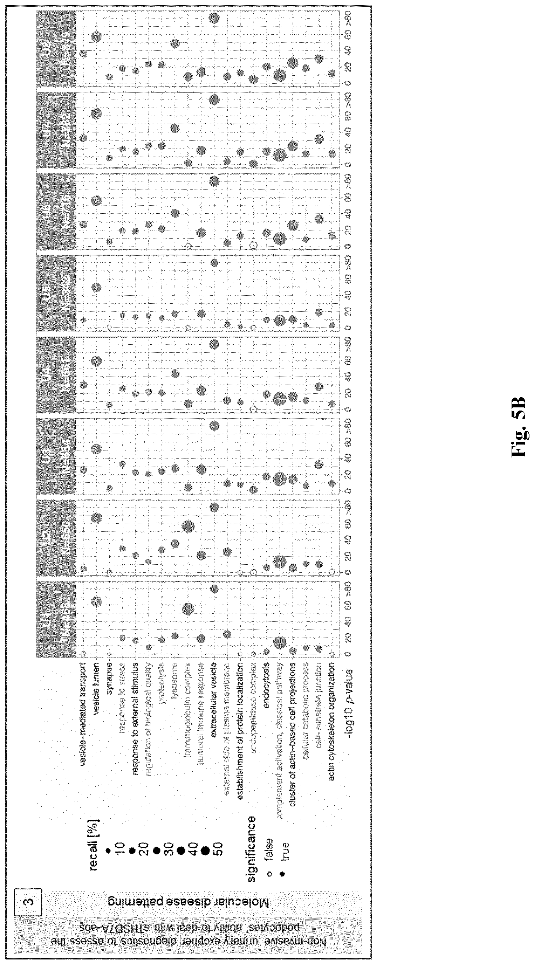

Figure 5B. *Panel 3:* Proteomic molecular disease patterning of the exopher-enriched vesicle fractions (huIgG4 pulldown from 10⁷ total urinary EVs) of U1-U8. For pathway analysis, only proteins identified with ≥ 2 peptides with a Q-value < 0.001 were used. Graph displays the *p-*values corresponding to the significance of pathway enrichment of selected functions. *P*-values below 1E-80 were capped; recall rate = proteins identified and assigned to category / number of proteins that are annotated to the category. In addition to the exopher proteins, some blood related proteins were found in high abundance as a result of the patient's nephrotic range proteinuria.

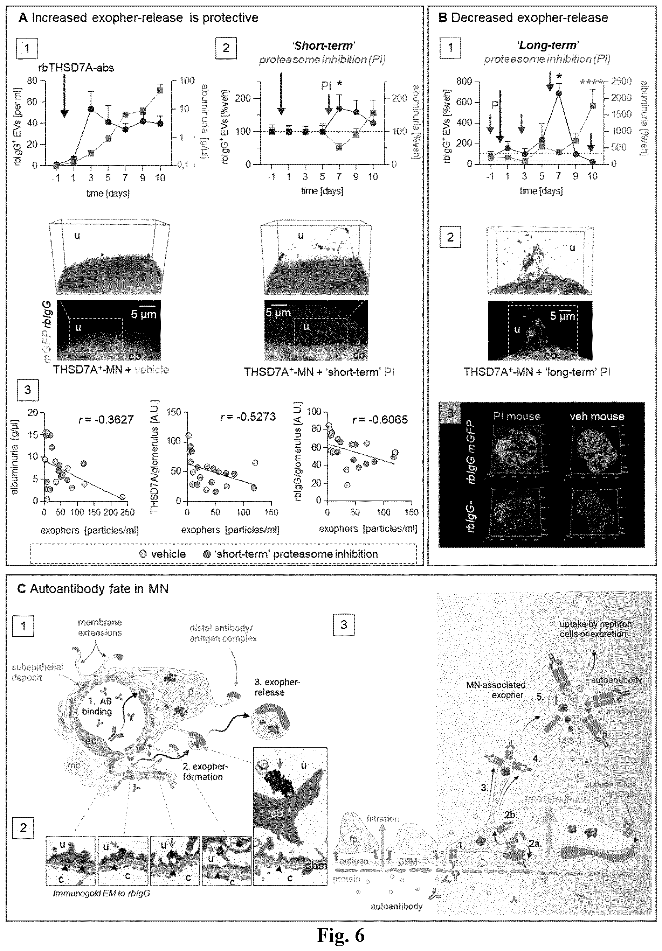

Figure 6. Protective exopher-release in experimental THSD7A⁺-MN.

A) *Panel 1:* THSD7A⁺-MN was induced in *mT*/*mG* reporter and BALB/c mice on day 0 by administration of rbTHSD7A-abs (black arrow). Abundance of rbIgG⁺-EVs was assessed from 10⁹ total urinary EVs by image stream; Graph depicts urinary exopher (rbIgG⁺-EVs; particles/ml) release (left *y* axis, black round symbols) and albuminuria (right *y* axis, grey square symbols) in THSD7A⁺-MN. *Panel 2:* Short-term proteotoxic stress was achieved by administration of a proteasomal inhibitor (epoxomicin, gray arrow) or vehicle (DMSO) every day after day 7 for 4 consecutive days. Graph depicts changes in urinary exopher release (left *y* axis, black symbols) and albuminuria (right *y* axis, grey symbols) in short-term proteasome inhibited relative to vehicle-THSD7A⁺-MN mice (dashed lines at 100%); pooled values from 3 independent experiments; *N* > 9 per group, mean ± SEM; **p* < 0.05, 2-way ANOVA with Tukey's multiple comparison test. On day 10 glomeruli were isolated, kidney slices of *mT*/*mG* mice that express membrane (m) bound GFP (mGFP, light grey) in podocytes were optically cleared and stained for bound rbTHSD7A-abs (dark grey). 3D-reconstructed z-stacks of the podocyte cell body (cb) plasma membrane are shown; u = glomerular urinary space. *Panel 3:* Spearman's correlations of *left graph* albuminuria (g/µl) and respective urinary exopher abundance (particles/ml); *middle & right graphs* of total glomerular THSD7A or rbIgG heavy chain protein abundance to the respective urinary exopher abundance (particles/ml) at time of sacrifice. B) Long-term proteotoxic stress was achieved by administration of the proteasomal inhibitor (bortezomib, grey arrows; (*N* = 6) or vehicle (DMSO; (*N* = 4) prior to MN induction on day -1 followed by 2 times per week for 10 days; 1 experiment. *Panel 1:* Graph depicts changes in urinary exopher release (left *y* axis, black round symbols) and albuminuria (right *y* axis, grey square symbols) in long-term proteasome inhibited relative to vehicle THSD7A⁺-MN mice (dashed lines at 100%); values from 1 experiment; N > 4 per group, mean ± SEM, *****p* < 0.0001, 2-way ANOVA with Tukey's multiple comparison test. *Panel 2:* On day 10, kidney slices of an *mT*/*mG* mouse that was optically cleared and stained for bound rbTHSD7A-abs (dark gray). A 3D-reconstructed z-stack of the podocyte cell body (cb) plasma membrane is shown; u = glomerular urinary space. *Panel* 3: Confocal microscopy of GFP (grey) and rbIgG (white) on cleared kidney slices of THSD7A⁺-MN mice with long term proteasome (PI) or vehicle (veh) administration; C) Concept of autoantibody fate using THSD7A as model antigen. *Panel 1:* The glomerular autoantibody burden depends on 1. the amount of circulating autoantibody that binds subepithelially to THSD7A and the ability of podocytes to dispose of these autoantibodies by 2. exopher-formation and 3. release to the urine. *Panels 2 & 3*: Exopher-formation involves autoantibody mediated THSD7A crosslinking in the subepithelial space (1.), deposition of THSD7A/autoantibody complexes in the subepithelial space (2a., classical concept of deposit formation; black arrowheads in the immunogold EM micrographs) and/or translocation towards the apical foot process membrane (2b., new concept; grey arrows in the immunogold EM micrographs). Apically displaced THSD7A/autoantibody complexes at foot, major processes and podocyte cell body extend to the urinary space through exopher-formation (3.). Exophers contain THSD7A and the extracellularly bound autoantibodies in addition to 14-3-3 as discerning EV marker, disease associated proteins, dysfunctional mitochondria, and degradative machinery (4.). Exophers are released to the urine and can be taken up by other nephron cells (5.). Scheme created with Biorender; ec = endothelial cell, mc = mesangial cell, p = podocytes, GBM = glomerular basement membrane, fp = foot process.

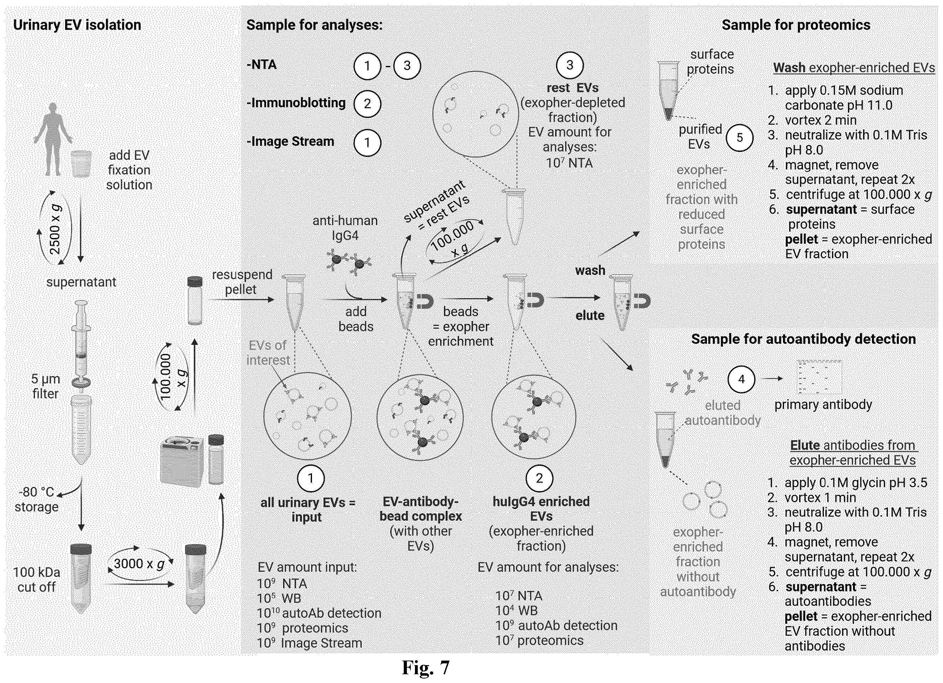

Figure 7. General workflow of EV sample preparation from patient urine as detailed in the method section. Illustration was created with Biorender.

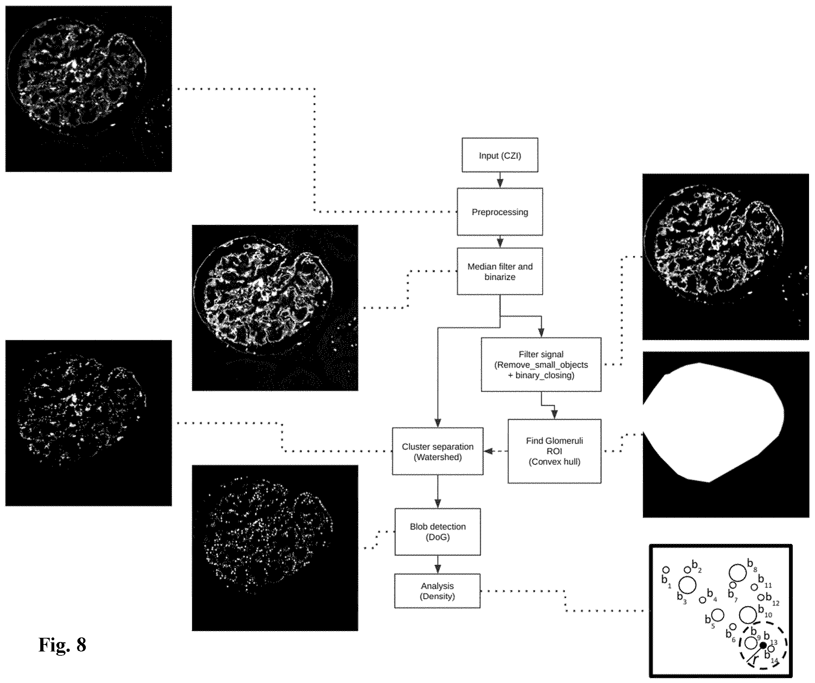

Figure 8. Computational image analyses of PLA₂R1 aggregate density. The algorithm was implemented in Python 3, and algorithm inputs were images saved in Carl Zeiss Image (CZI) Data file format. Grayscale images were converted to binary images and further processed for blob detection. Based on detected blobs the PLA₂R1 density for each blob is calculated. The PLA2R1 aggregated density per patient is calculated as the median of all PLA₂R1 densities of all images of that patient. The bottom depicts an example calculation of the density for a blob b₁₃. In the radius (r) around the blob are two other blobs present (b₉ and b₁₄). A total of 14 blobs are present in the example. The density of b₁₃ is therefore 2/14 = 0,1429.

### EXAMPLES

In comparative studies to nephrotic non-MN patients, glomerular aggregates as disease-specific exophers in MN were established. The podocytes' ability to form and release exophers to the urine was assessed in retrospective and prospective patient cohorts, and its additive clinical value in disease monitoring were shown. Using experimental podocyte and mouse models of THSD7A⁺-MN the dynamics and disease modulating effects of exopher formation and release was investigated.

### Materials and Methods

***Abbreviations:*** IS = image stream; IF = immunofluorescence; WB = immunoblot; iEM = Immunogold EM; PD = Pulldown; A = application; rb = rabbit, ms = mouse; gt = goat; ha = hamster; gp = guinea-pig; dk = donkey, mAb = monoclonal antibody, o/n = over night

### Cell culture

Human podocytes: Human immortalized podocytes (a kind gift of Moin Saleem, University of Bristol, Bristol, Great Britain) were cultured under permissive conditions [32 °C, 5% CO₂, RPMI 1640 Thermo Fisher, #61870-010 supplemented with 10% fetal calf serum, 1X insulin, transferrin selenium (ITS, PAN Biotech, #P07-03200), 1 µg/ml Puromycin (Sigma, #P7255)] in uncoated, vented tissue culture flasks (Sarstedt) as described [63]. Stable overexpression of human THSD7A with a C-terminal myc-Flag tag was achieved by lentiviral transduction. Mock cells were transduced with an empty vector [32]. For differentiation, podocytes were cultured for 10 days under non-permissive conditions [37°C, 5% CO₂, RPMI 1640 supplemented with 10% FCS, 1X ITS]. Cell density was kept below 80-90% to allow process development. DNA was extracted from cell pellets and controlled for the absence of mycoplasma infections on a 2-3 monthly basis according to the manufacturer's instructions (Minerva Biolabs #11-8100), cellular passages below 60 were used for experiments. To collect extracellular vesicles, podocytes were transferred to culture in an exosome-depleted medium (Thermo Fisher, #A2720803) 16 hours prior to commencement of antibody treatment. In vitro antibody treatment was performed with human or rabbit anti-THSD7A-specific antibodies or unspecific control huIgG or rbIgG, all 1:200 from a 4 mg/mL stock solution) for 0, 1, 6, 24, and 48 hours. Podocytes were harvested by scratching in PBS on ice. Cell pellets were collected and lysed in T-PER (Thermo Fisher, #78510) with SigmaFast EDTA-free protease inhibitor cocktail (Sigma, #S8830). Extracellular vesicles (EVs) were collected from 240.000 cells in 3 ml exosome-depleted medium.

HEK293T cells: HEK293T cells (Sigma, # 12022001) were cultured in DMEM supplied with 10% FCS and 1X penicillin/streptomycin (Thermo Fisher, #15070063) at 37°C and 5% CO2. Transient transfection of HEK293T cells with full length human (hu)THSD7A (Origene, #RC213616) or huPLA₂R1 ([3)] constructs was performed using JetPEI (VWR, #101000053) according to the manufacturers' instruction. For that purpose, 5 µg of respective plasmid DNA was mixed with 10 µl of JetPEI and 10 mM sodium chloride in a final volume of 500 µl and after incubation for 25 minutes at room temperature gently added to < 80% confluent HEK293T cells that were seeded at the same time day of transfection and allowed to attach to the bottom of a 6 cm cell culture plate (Sarstedt, #83.3901). Cells were allowed to express the protein for 48 hours at 37°C and 5% CO2. Cells were harvested by scraping them with a rubber policeman in ice cold PBS (Thermo Fisher, #14190-169). The resulting cell suspension was transferred to a 1.5 ml Eppendorf tube, centrifuged at 4°C at 900 x g for 10 minutes and the supernatant was discarded. To remove remaining medium residues, the pellet was resuspended in PBS, centrifuged again at 4°C at 900 x g for 10 minutes, supernatant was discarded, and the pellet were lysed in T-PER (Thermo Fisher, #78510) after addition of Proteinase Inhibitor Cocktail (Sigma, #S8820) and PhosStop (Sigma, #4906845001). After protein quantification by the use of ROTI^{®}Quant universal (Carl Roth, #0120.1) according to the manufacturers' instruction, 10 µg of total protein was prepared for immunoblotting under non-reducing conditions (loading buffer as described below but without DTT) for detection of autoantibodies to huTHSD7A or huPLA₂R1 in murine sera or in the eluted antibody fraction from enriched patient exophers.

### Phase contrast live cell imaging

Podocytes were seeded in 35 mm diameter bottom glass fluorodishes (World Precision Instrument, WPI) coated with Poly-L-Lysine. Imaging was performed with a Leica DMi8 M/C/A inverted microscope using a HC PL Fluotar 20x/0,4 CORR PH1 objective (Leica Microsystems). Podocytes were imaged for 4 hours; treatment was started 30 minutes prior to commencement of imaging with addition of rbTHSD7A-abs or ctrl-abs to the medium. The images were recorded with an ORCA-Flash4.0 digital camera (Hamamatsu Photonics), and Meta-Morph^{®} Version 7.10.5.476 software (Molecular Devices). Pictures were acquired every 1 min for a total duration of 4 hours. Movies were reconstituted, processed, and analyzed using Fiji (ImageJ 1.53t) software. The tracking was performed using the TrackMate v7 plugin from Fiji [75, 19] after processing the movies as previously described [62]. During the acquisition, podocytes were kept at 37°C, and 5% CO₂ inside a humid chamber on the objective stage.

### Holotomography

Refractive index detection and holotomographic images were acquired in a Nanolive 3D Cell Explorer-fluo (Nanolive SA, Tolochenaz, Switzerland), equipped with a 60x/0.8 objective, and STEVE software (Full v1.6.3496), as previously described ([64]). Podocytes were imaged 45 minutes after treatment with rbTHSD7A-abs or ctrl-abs as described in the previous sections. For the acquisition, the cells were kept at 37°C, and 5% CO₂ inside a humid chamber on the objective stage. The holotomographic images were processed by using the post-processing plugins in STEVE. According to the manufacturers' instructions the following image processing was performed: first a histogram a log10 adjust was made, followed by a background subtraction (automatic estimation). No edge preserving filter was applied. The 3D images of the refractive index were made later in Fiji (Imaged) by using the plugin 3D project.

### Animal experiments

Male BALB/c mice with a pure genetic background were purchased from Charles River, Gt(ROSA)26Sortm4(ACTB-tdTomato,-EGFP)Luo/J x hNPHS2Cre mice (mT/mG) [11] with a BALB/c background were provided from the Huber laboratory (III Medical Clinic, UKE, Hamburg-Eppendorf) and were predominantly analyzed at 16-22 weeks of age. mT/mG reporter mice express membrane-bound GFP in podocytes and all other renal cells express membrane-bound tdTomato. Mice had free access to water and standard animal chow (Altromin 1328 P), were synchronized to a 12:12 hour light-dark cycle and were housed in a pathogen-free animal facility at the University Medical Center Hamburg-Eppendorf. This study focused on THSD7A turnover, which contrasting PLA₂R1, is constitutively expressed in rodents ([48]), and reliable mouse models of THSD7A⁺-MN have been developed [67, 78]. In the passive model of THSD7A⁺-MN ([78]), mice are exposed to rabbit anti-THSD7A antibodies (rbTHSD7A-abs) by intravenous injection. These rbTHSD7A-abs bind to the large extracellular region of THSD7A as a type 1 transmembrane protein with a single transmembrane domain, and a short intracellular domain ([76]). Similarly to the patient autoantibodies to THSD7A, rbTHSD7A-abs exhibit a binding affinity to THSD7A that is sensitive to the conformation of the protein [78, 66]. THSD7A localizes to the most basal aspect of the podocyte foot process domain near the slit diaphragm underneath Nephrin [32]. Accordingly, autoantibody binding rapidly occurs at the subepithelial aspect of FPs, preferentially at the slit diaphragm ([32]) and mice develop proteinuria after 1-3 days, which reaches nephrotic ranges after 7 days. In this model, glomerular accumulations of THSD7A and rbTHSD7A-abs are observed in a comparable pattern to patients with THSD7A⁺-MN ([78]), albeit to a lesser degree.

Here, THSD7A⁺-MN was induced by intravenous injection of 180 µl (1,5 mg/ 30 g mouse) rabbit anti-human/mouse THSD7A antibodies or unspecific rabbit IgG as a control in male BALB/c or mT/mG mice aged 12-22 weeks ([78]). For 'short-term' inhibitor studies mice were treated on four consecutive days with either the proteasomal inhibitor epoxomicin (0.5 mg/kg bodyweight in 125 µl, Enzo, #BML-PI127-0100) or vehicle (25% DMSO in 125 µl, Carl Roth, #4720.2) by intra-peritoneal injection. For 'long-term' proteasome inhibitor studies, treatment with the proteasomal inhibitor bortezomib (0.5 µg/g bodyweight in 125 µl DMSO; PS-341, UbpBio, #F1200) or vehicle (25% DMSO in 125 µl, Carl Roth, #4720.2) was commenced one day prior to THSD7A-MN induction by intra-peritoneal injection 2 times/ week for 11 days. Animal euthanasia was performed following subcutaneous buprenorphine hydrochloride (0,1 mg/kg KG, TEMGESIC, Indivior Europe Limited, #ind00979) administration for analgesia 30 minutes prior to cervical neck dislocation under 3.5% isoflurane (Baxter, #HDG9623) inhalation narcosis. During all procedures, body temperature was kept at 37 ± 0.5°C. All experimental procedures were performed according to the institutional guidelines.

### Sample collection

For sample collection, mouse urine was collected in metabolic cages over a period of 4 hours. Isolated kidney packages were thoroughly perfused through the renal arteries with 5 ml PBS/DynabeadsTM M-450 Tosylactivated (Invitrogen, Thermo Fisher, #4013) per kidney to remove blood components such as unbound immunoglobulins from the renal circulation. Successful perfusion was controlled by the color of the kidney. Samples were stored at -80°C prior to further analysis, fixed in 4% PFA for histological evaluations, or processed for glomerular isolation.

### Mouse serum analysis

Mouse blood was centrifuged for 10 min at 1500 x g, 4°C to separate blood cells from serum. In serum circulating specific rabbit THSD7A antibodies were detected by immunoblotting. For this, huTHSD7A and huPLA₂R1 protein produced in HEK cells was loaded non reduced on a SDS-PAGE. Mouse serum (300 µl) was mixed 1:2 with SuperBlockTM (Thermo Scientific #37515), incubated as primary antibody on the WB membrane over night at 4°C and detected with anti-mouse HRP secondary antibody.

### Urinary albumin to creatinine ratio

Urine albumin content was quantified using a commercially available ELISA system (Bethyl, Montgomery, USA, #A90-134A) according to the manufacturer's instructions. Briefly, 96-well plates were coated 1:100 with goat anti-mouse albumin in binding buffer (0.05 M carbonate-bicarbonate pH 9.6) over night at 4°C. After washes in 50 mM Tris, 0.14 M NaCl, 0.05% Tween-20 pH 8.0, the plates were blocked for 30 min at room temperature with 50 mM Tris, 0.14 M NaCl, and 1% BSA pH 8.0 and rewashed. Diluted urine was incubated for 60 min at RT. Following washes, the secondary antibody (1:40,000; horseradish peroxidase goat anti-mouse albumin) was applied for 60 min at room temperature. After washes, enzyme substrate supplied by the kit was added and the color development was stopped after 5 min with 2 M phosphoric acid. Extinction was measured at 450 nm in an ELISA plate reader (BioTek, #EL 808). The urinary albumin concentration was calculated according to the formula for absorption = (A - D)/1 + (x/C)B + D, where A and D are values from the standard curve. Regression values for the standard curve were calculated to assess the accuracy of the measured values. Standard curves with r values > 0.9950 were used. Urinary albumin values were standardized against urine creatinine values of the same individuals determined by Jaffé (Hengler analytic, #114444) or to the urine volume and plotted as [g albumin / g creatinine] or as [g albumin / µl urine].

### Glomeruli isolation

Murine glomeruli were isolated using Dynabead perfusion as described [74]. In brief, kidneys were perfused with 5 ml magnetic bead solution per kidney (magnetic bead solution: of 50 µl Dynabeads^{™}; M-450 Tosylactivated Invitrogen ThermoFisher #14013 with 200 µl SPHERO Polystyrene Magnetic Particles, Spherotech, #PM-40-10 diluted in 50 ml HBSS) and digested with collagenase (in HBSS, 1.2 mg/ml collagenase 1A, Sigma, #C9891, 100 U/ml DNaseI, Sigma, #10104159001) for 15 min, 37°C, 1.300 rpm. The solution was strained through a 100 µm cell strainer (Sarstedt, #83.3945.100), which was rinsed with HBSS, this was done 3 times. The solution was centrifuged for 5 min, 600 x g, 4°C. The pellet was resuspended in HBSS, and the glomeruli were separated from tubuli with a magnetic particle concentrator. The number of isolated glomeruli and contaminating tubuli was quantified under the phase contrast microscope prior to storage of glomeruli at -80°C.

*Human glomeruli:* Human glomeruli were used as positive controls for immunoblots. Healthy parts of kidneys from patients who underwent nephrectomy were used for the preparation of an extract of human glomeruli. Cortex was separated from the medulla, the cortical tissue was weighed and pulpified with a scalpel. Approximately 0.5 g tissue was portioned into each reaction tube and digestion solution (1.2 mg/ml Collagenase 1 A, 100 U/ml DNase I) was added. Digestion was performed for 15 min, 37°C, 1.300 rpm in a ThermoMixer (Eppendorf). The tissue homogenate was sieved through a 212 µm sieve placed on a beaker and pushed through with a small Erlenmeyer flask using low pressure. In between the tissue was rinsed multiple times with PBS containing 0.05% BSA. To finally separate glomeruli from tubular remnants, 53 µm sieves were used whereby intact glomeruli remained on the sieve. By using a syringe filled with PBS containing 0.05% BSA, a small cannula, and high pressure the glomeruli were flushed into a clean beaker. To increase the purity grade of the glomerular isolate, the last step was performed two times, using clean 53 µm sieves each time. The collected suspension was centrifuged for 10 min at 800 x g, 4°C. The supernatant was decanted, and the pellet was solved in PBS containing 0.05% BSA. The number and purity of isolated glomeruli was determined under a phase contrast microscope.

### Morphological analyses

Cleared kidney slices: Kidney pieces from mT/mG mice were immersion fixed with 4% PFA o/n at 4°C and sliced 300 µm thick with a Vibratome (VT1000S, Leica Biosystems Nussloch GmbH, Germany), washed with PBS and optically cleared for 48 hours using SCALEVIEW A2 (FUJIFILM Wako Chemicals, #193-18455). For immunofluorescence of exophers, cleared slices were incubated with cy5 anti-rabbit IgG (1:100 for 3 days). After extensive washing over 48 hours, 3D image stacks were acquired using a Nikon A1 R confocal laser scanning microscope with A 60x CFI Plan Apo Lambda immersion oil objective (NA 1.42). Additionally, a Visitron Spinning Disk Microscope equipped with a 100x CFI Plan Apo Lambda (NA 1.45) objective and a Yokogawa CSU W-1 Spinning Disk unit with 50 µm pinholes and a Photometrics Prime 95B (back-illuminated sCMOS camera) was employed. The mGFP and cy5 tags were visualized using solid-state lasers of 488, 640, and 647 nm and image stacks were recorded with 0.130 and 0.150 µm Z-steps, respectively. The 3D image deconvolution was carried out with Nikon's NIS-elements AR 5.42.02 using a theoretical PSF and employing an automatic deconvolution algorithm. 3D image data sets were subsequently further processed using Nikon Denoise.ai and Clarify.ai tools. Visualization of the 3D volume views of whole glomeruli as well as 2D MIPs from the podocyte cell body area were carried out using NIS-elements. 3D reconstruction of the image stacks in blend mode from the podocyte cell body area was rendered by utilizing Imaris 8.2.1. Brightness, contrast, and gamma were adjusted for optimal representation.

*Light microscopy:* For light microscopic evaluation, 1.5 µm thick paraffin sections were stained with periodic-acid-Schiff reagent (Sigma, #1.08033.0500) according to the manufacturer's instructions for the assessment of general renal morphology in the THSD7A⁺-MN patient. Nuclei were counterstained with hematoxylin (Serva, #24420.02).

*Immunohistochemical analyses:* 1 µM paraffin sections of the diagnostic renal biopsy of the THSD7A⁺-MN patient were deparaffinized and rehydrated. Antigen retrieval was obtained for THSD7A by boiling in DAKO antigen retrieval buffer, pH 9 (15 min at 98°C; DAKO, #S2367) and subsequent cooling and for huIgG4 and huIgG by protease XXIV (5 µg/ml; Sigma, #8038) for 15 minutes at 37°C and stopping of the reaction in 100% EtOH. Nonspecific binding was blocked with 5% horse serum (Vector, #VEC-S-2000) with 0.05% Triton X-100 (Sigma, #T8787) in PBS for 30 min at RT prior to incubation at 4°C o/n with rabbit anti-THSD7A, mouse anti-huIgG4, or goat anti-huIgG in blocking buffer. Staining was visualized with the ZytochemPlus AP Polymer kit (Zytomed Systems, Berlin, Germany, #POLAP-100) according to the manufacturer's instructions. Nuclei were counterstained with hematoxylin (Serva, #24420.02) and sections were mounted with gum arabic (Roth, #4159.3). Stainings were evaluated with an Axioskop using the Axiovision software (all Zeiss, Oberkochen, Germany).

*Immunofluorescent analyses:* Patient biopsy and mouse kidney paraffin sections were deparaffinized and rehydrated. Antigen retrieval was obtained by i) boiling in retrieval buffer, pH9 (#S2367, DAKO), pH6.1 (#S2369, DAKO) or 0.05% citraconic acid (#82604, Sigma) for 30 min at 98°C, followed by cooling or ii) protease XXIV (Sigma, #8038, 5 µg/ml) digestion 15 min at 37°C followed by a wash in 100% EtOH. Unspecific binding was blocked with 5% normal horse serum (#VEC-S-2000) in PBS 0.05% TritonX-100 (Sigma, #T8787) for 30 min at RT. Primary antibodies were incubated in blocking buffer o/n at 4°C. Following washes in PBS, fluorochrome-labeled donkey secondary antibodies (all 1:200, Jackson Immunoresearch Laboratories), biotinylated-WGA (followed by AF647-streptavidin) or rhodamine-coupled WGA, and Hoechst were applied where appropriate for 30 min at RT. After washes in PBS, sections were mounted in fluoromount (Southern-Biotech, #0100-01).

For immunofluorescence of urinary exophers, urinary EVs were stained for basic EV characterization using the image stream antibodies and according to the method described in the section image stream. For additional analyses of the exopher content, the staining panel was expanded to include the proteins of interest. Stained and washed EVs were airdried on poly-L-lysine (1:1000 in sterile water) coated slides and mounted with fluoromount. EVs were stained with MemBrite^{®}594/615, huIgG4-AF488 and 14-3-3-AF546 to demarcate exophers in combination with the proteins of interest.

For immunofluorescence of cultured podocytes, cells were extensively washed after live-imaging, fixed with 4% PFA for 8 min at RT. Following PBS washes, unspecific binding was blocked with 5% normal horse serum (#VEC-S-2000) in PBS 0.05% TritonX-100 (Sigma, #T8787) for 30 min at RT. Primary antibodies were applied o/n in blocking buffer at 4°C. The following day, samples were PBS washed and incubated with secondary antibodies (all 1:200) in blocking buffer for 30 min. Samples were PBS washed and mounted with fluoromount. Immunofluorescence was resolved by conventional and high-resolution confocal microscopy either with a LSM800 with Airyscan 1 or with a LSM980 with Airyscan 2 microscope using ZEN 3.0 software (all ZEISS).

### Computational image analysis

35 patient biopsies of a retrospective PLA₂R1⁺-MN patient cohort [57] were stained for PLA₂R1. Each glomerulus present within the biopsy cylinder was imaged using an LSM800 in confocal modus at a 2873 x 2873 resolution, Plan-Apochromat 63x/ 1.40 Oil DIC M27 objective, pinhole 1AU. The algorithm was implemented in Python 3 (mainly packages Scikit-image, Numpy and OpenCV) using a Difference of Gaussian blob detection [81]. Algorithm inputs were images in Carl Zeiss Image (CZI) Data file format. Using the grayscale PLA₂R1 channel, images were, in a first step, preprocessed to bring the images into the same representation space and make individual patients comparable. The steps for the preprocessing were as follows, first, images were percentile normalized (2% as lower bound and 98% as upper bound) to accommodate for the varying signal distributions within the data. This step is carried out because, depending on where the Glomerulus was sliced, the amount of signal in the images varied and caused the histogram of the image to look different. Second, to make the algorithm robust against different parameters used during image acquisition, the resolution is sampled to a resolution of 0.049 micron per pixel. This ensures that features withing the images have comparable sizes. After preprocessing, for the identification of the exophers, the preprocessed images were median filtered and converted to binary images by using a Multi-Otsu approach [43], separating high signal areas of the exophers from background noise. The thresholding approach used still left smaller artificats in the binary image which were filtered out by using morphological operators. The binary image was therefore further processed by removing small objects with size smaller 40 pixels and applying the morphological operation of closing with a size of 60. Based on the now cleaned binary image a glomerular mask was extracted by calculating the convex hull around the binary signal and therefore roughly following the Bowman's capsule or the glomerulus outline. In the masked area signal clusters (areas of overlapping or touching signal blobs) were separated from each other using a watershed transformation [50]. In the further step, the binary output of the watershed separation is used as input for the Difference of Gaussian (DoG) blob detection [81]. The resulting detected blobs were considered exopher candidates.

For the following analysis the detected exopher candidates's number, size, image intensity and centroids (geometric center of each exopher) were saved. Based on the detected blobs the PLA₂R1 density for each blob is calculated. PLA₂R1 density is defined as the sum of the number of neighboring blobs (excluding the current blob) within a radius of 50 pixels around each blob divided by total number of blobs in the image. The division normalizes the image to a range from 0 (all blobs have 0 neighbors) to the total number of blobs (all blobs have all blobs as neighbors). The PLA₂R1 aggregated density per patient is calculated as the median of all PLA₂R1 densities of all images of that patient.

### Ultrastructural analyses

*Pre-embedding Immunogold EM*: Experimental mice were in vivo fixed for immunogold labeling modified according to Somogyi and Takagi (1982) [71]. In brief mice were transcardially perfused with 0.1 M phosphate buffer (PB) pH 7.4 at 4°C. One liter fixative consisted of 500 ml 0.2 M PB pH 7.4, 150 ml saturated picric acid (Bouin's solution: 0.044 M picric acid, 5% (V/V) acidic acid, and 10% (V/V) formaldehyde in aqua dest.), 346.8 ml paraformaldehyde solution (40 g PFA in aqua dest.) and 3.2 ml of 25% glutaraldehyde at a final pH 7.4. The final concentrations were 4% paraformaldehyde, 0.08% glutaraldehyde, and 0.15% picric acid. Kidneys were removed after perfusion procedure and postfixed in 100 ml 0.1 M PB including 4% paraformaldehyde and 0.15% picric acid for 24 hrs at 4°C. Vibratome sections (LEICA VT 1000S) of fixed tissue were made with a thickness of 60 µm of areas of interest and the slices were placed into cryoprotection solution containing 0.1 M PB (pH 7.4), 30% sucrose, 1% polyvinylpyrrolidone, and 30% ethylenglycol. For pre-embedding immunogold staining, cryoprotection solution was removed from the 60 µm thin slices by washing twice in 0.1 M sodium phosphate buffer pH 7.4 (PBS) for 20 minutes, followed by washing of the slices in 0.1 M PBS with 10% sucrose for 30 minutes and 0.1 M PBS with 20% sucrose for further 30 minutes. Tissue was transferred to a drop of PBS with 20% sucrose and frozen briefly with nitrogen and thawed to room temperature. Subsequently, the tissue was washed three times in PBS pH 7.4 for 10 minutes. Blocking reagent solution (BRS) were prepared with 100 ml PBS, 800 mg BSA (Sigma-Aldrich A7638-5G), 250 µl 40% cold water fish skin gelatine (AURION CWFSG 900.033) and 162.5 mg sodium acid. For detection of bound rbTHSD7A-abs, 12 nm Colloidal Gold-AffiniPure Donkey anti-rabbit IgG (H+L) (Jackson ImmunoResearch) was used. For the detection of THSD7A antigen, indirect immunostaining was performed, using the affinity-purified, pathogenicity-inducing patient THSD7A autoantibody [77] as primary antibody to omit the risk of cross-reactivity to the injected and bound rabbit THSD7A-abs and to the intrinsic mouse IgG within the sample. The bound patient THSD7A autoantibody was then detected by 12 nm Colloidal Gold-AffiniPure Goat anti-human IgG (H+L) (Jackson ImmunoResearch). To increase the specificity of primary antibody reaction, blocking reagent solution (BRS) supplemented with 2% normal goat serum (NGS, Sigma) was used for one hour for blocking reaction. The primary antibody was solved in BRS overnight at 4°C. After 24 hours the tissue was washed in PBS 3 times for 5 minutes each and incubated with 12 nm gold conjugated antibody solution (1:50) in BRS for 2 hours. Subsequently, the tissue was washed three times in PBS pH 7.4 every 5 minutes, followed by 1% glutaraldehyde solution in 0.1 MPB for 10 minutes. The tissue was solved in 1% OsO4 in aqua dest. at 4°C for 10 minutes followed by dehydration in an ethanol series. Subsequently, the probes were transferred twice into propylene oxide for 5 minutes and embedded in a mixture of epon and propylene oxide (4: 1) overnight. Tissue samples were cut with an ultra-thin (Reichert Jung Ultracut) microtome and thin sections were inspected in a Zeiss electron microscope (TEM Leo 910) and TEM Jeol 1400plus. Pictures were taken assisted by ImageSPViewer (Tröndle) and image viewer Jeol.

*3D reconstruction:* 3D reconstruction of 13-48 consecutive EM micrographs was performed using the HiD^{®} Histodigital reconstruction approach [27]. First, the orientation of the micrographs was homogenized using rigid image registration, such that all images were aligned. Since the thin sections are deformed during cutting and further processing, they are affected by non-linear deformations which prevent a coherent 3D impression even after slice alignment. Therefore, in a second step, an intensity-based non-rigid registration routine is applied to reverse these deformations such that spatial coherence of the data set is achieved. Volume visualizations were created with the Chimaera SDK.

*Scanning electron microscopy (SEM):* For evaluation in SEM, bouin fixed 60 µm mouse kidney slices were incubated in series of ethanol/aqua dest. bath with increasing ethanol concentration (30%, 50%, 70%, 90%, 100%,) at ten minutes intervals followed in acetone/EtOH bath by increasing acetone concentration (50%, 70%, 90%, 100%) at ten minutes intervals. Then tissue probes were dried in critical point dryer (Bal-Tec CPD 030) and stored in Vacuum dryer. The dried tissue probes were placed with conducting silver on carbon plate on a metallic probe holder and dried again in vacuum chamber for 24 hours. The surface was sputtered by 5 nm thin platinum layer in a sputter coater (Bal-Tec SCD 500) and evaluated on SEM Jeol 7500F via YAG and LABE Backscatter electron detector (BSE).

### Extracellular vesicle (EV) isolation

*EV isolation from human podocyte culture:* Medium was collected from cells and fixation solutions were added to the collected cell culture medium [1:100; solution 1: 660 mM NaN3 (Sigma, #S2002), 200 mM EGTA (Carl Roth, #3054.2) in ddH₂O, solution 2: 100 mM PMSF (Merck-Millipore, #P7626) in 100% ethanol]. The medium was centrifuged for 10 min, 2.000 x g, 4°C, the supernatant was removed and centrifuged for 10 min at 2.000 x g and 4°C. The supernatant was ultra-centrifuged for 1.5 hours at 100.000 x g and 4°C, the supernatant was discarded, and the pellet resolved in filtered PBS.

*EV isolation from mouse urine:* Fixation solution was added to the collected urine [1: 100; solution 1: 660 mM NaN3, 200 mM EGTA in ddH2O, solution 2: 100 mM PMSF in 100% ethanol]. Urine samples were centrifuged for 10 min at 5.000 x g and 4°C. To reduce the protein amount of the nephrotic urine the supernatant was dialyzed for 20 hours in 1 × PBS at 4°C on a magnetic stirrer, using a 100 kDa cut-off dialysis membrane Spectra/Por Biotech CE (Carl Roth, #8967.1). The dialyzed urine was ultra-centrifuged for 1.5 hours at 100.000 x g and 4°C. The supernatant was discarded, and the pellet solved in filtered PBS. Concentration was measured with MemBrite^{®} Fix Cell Surface dye 594/615 by Image Stream.

*EV isolation from patient urine:* The general workflow for total urinary EV isolation and subsequent huIgG4⁺-EV enrichment for the individual analyses (image stream, immunofluorescence, immunoblotting, immunogold EM, NTA, autoantibody elution, proteomics) is summarized in Supplementary Fig. 7. Nephrotic patients admitted to the clinic for a diagnostic kidney biopsy were included to the study. Prior to kidney biopsy, a 24h urine or spot urine was collected and processed as follows. Fixation solution was added to the collected urine [1:100; solution 1: 660 mM NaN3, 200 mM EGTA in ddH₂O, solution 2: 100 mM PMSF in 100% ethanol] and centrifuged for 10 min at 2.500 x g, 4°C. The supernatant was filtered through 5 µm syringe filters (Millex Merck Millipore, #SLSV025LS) to remove cell debris. 50 ml aliquots were stored at -80°C prior to use. Patients were included to the study after serological and nephropathological diagnosis of following diseases: THSD7A⁺-MN (Patient (P)1), PLA₂R1⁺-MN (P2-P4), minimal change disease (P5, P6), IgA nephritis (P7), tubulo-toxic kidney injury (P8), and primary focal segmental glomerulosclerosis (pFSGS: P9, P10). Two patients (P1 and P2) were prospectively followed, and urinary samples were collected at time of their routine clinical management. Stored urine samples were thawed over night at 4°C, thoroughly vortexed and centrifuged for 10 min at 2500 x g, 4°C to remove aggregates. To reduce the abundance of contaminating proteins from the highly proteinuric urine, the urine was ultra-filtrated by centrifugation at 3.000 x g and 4°C using 100 kDa cut-off filter tubes (Amicon Ultra-15 Centrifugal Filter Unit, Merck-Millipore, #UFC910024), until half of the initial volume was reached. The filtered supernatant was ultra-centrifuged for 1.5 hours, 100.000 x g and 4°C, the supernatant was discarded, and the EV pellet was solved in filtered PBS. Particles/ml were measured with MemBrite^{®} Fix Cell Surface dye 594/615 by image stream. The resulting EV samples are called total EVs (input) in the following methods (= sample 1, Fig.7).

### Enrichment of huIgG4⁺-EVs from total EVs

*Preparation of huIgG4-beads for EVpulldown:* Dynabeads^{™} M-450 Tosylactivated (Invitrogen ThermoFisher, #14013) were washed with 0.1M sodium phosphate buffer pH 8 on a DynaMag. Anti-human IgG4 antibody (Mouse Anti-Human IgG4 pFc'-UNLB, Southern-Biotech, #9190-01) was coupled to Dynabeads according to manufacturers' instructions. Antibody coupled beads were blocked 1:1 with 2% cold fish gelatine (Sigma, #G7041) and SuperBlockTM (Thermo Scientific, #37515) in filtered PBS for 1 hour at room temperature on a roller shaker. Beads were washed on a DynaMag twice with 0.1M sodium phosphate pH 8.

*HuIgG4⁺-EV pulldown*: Depending on the planned assay, 105 (immunoblotting), 109 (proteomic analyses), 109 (image stream, NTA), or 1010 (autoantibody elution) total EVs (= sample 1, Suppl. Fig. 21) were used as starting material. Total EVs were added to blocked huIgG4-beads, filled up to 250 µl with 2 mM EDTA in filtered PBS pH 7.4 and incubated overnight on a roller shaker at 4°C. EVs bound to huIgG4-beads were concentrated on a DynaMag. Enriched (bead bound) huIgG4⁺-EVs (= sample 2 in Fig.7) were eluted from the beads for quantification depending on the planned analyses. In general, EV concentration (particles/ml) was measured by image stream using MemBrite^{®} Fix Cell Surface dye 594/615. The supernatant containing the rest EV fraction (huIgG4⁺-EV depleted) was removed and processed by ultra-centrifugation for 1.5 hours at 100.000 x g and 4°C to pellet the rest EVs (= sample 3 in Fig. 7). The rest EV pellet was dissolved in sterile PBS, concentration (particles/ml) was measured by image stream as detailed above.

### Image Stream

Vesicle abundances and marker composition were analyzed by image stream. Following starting material was used: 1) podocyte EVs released into 3 ml of exosome-depleted medium, 2) total EVs isolated from 250 µl of mouse urine 3) 109 EVs isolated from patient urine. To determine the particle/ml concentration of individual EV sample preparations, 3 µl were stained.

*EV staining for image stream:* EVs were stained in filtered PBS containing 8% exosome-depleted FBS (Thermo Fisher, #A2720803) in an end volume of 10 µl and stained for 45 min in the dark at room temperature. Stained EVs were washed in 500 µl 2% exosome-depleted FBS in filtered PBS using 300 kDa filter (PALL Nanosep Centrifugal Devices with Omega Membrane - 300K), centrifuged for 10 min at 5000 x g and resolved in wash buffer containing 2% exosome-depleted FCS in filtered PBS. Controls included for all analyses were buffer controls without EVs and antibodies, unstained EV samples, and buffer with antibodies only. All antibodies used for image stream were centrifuged for 10 min at 15.000 x g at 4°C before use. Antibodies used to stain murine samples were cy5 anti-rabbit (Jackson ImmunoResearch Laboratories), AF546 anti-14-3-3, CoraLite Plus 647 anti-AnnexinV (Proteintech), and 1x MemBrite^{®} Fix Cell Surface dye 594/615 (Biotium). For human EVs staining the antibodies used were, CoraLite^{®} 488 anti-human IgG4 (Proteintech), PacBlue anti-CD63 (BioLegend), PacBlue anti-CD81 (Biolegend), APC anti-AnnexinA1 (Novus Biologicals), CoraLite Plus 647 anti-AnnexinV (Proteintech) and 1x MemBrite^{®} Fix Cell Surface dye 594/615 (Biotium).

*Image stream measurements:* EVs were analyzed on an ImageStreamX MkII (ISX Amnis/MilliporeSigma) with a 60x magnification and low flow rate with standard sheath fluid (Dulbecco's PBS pH 7.4). mGFP, FITC, and cy2 signal were collected in channel 2 (480-560 nm filter), mTomato and AF546 signal in channel 3 (560-595 nm filter), MemBrite^{®} Fix Cell Surface dye 594/615 signal in channel 4 (595-640nm filter), PacBlue signal in channel 7 (430-505 nm filter) and AF647, cy5 and APC signals in channel 11 (640-745 nm filter). Channels 1 and 9 were used as brightfield channels (430-480 nm and 570-595 nm filter) and channel 6 (745-800 nm filter) for site scatter (SSC) detection. Mouse EV gating: For mT/mG reporter mice all fluorescent EVs positive for mTomato (Ch03) and mGFP (Ch02) were used as total vesicle population. Gates only positive for mGFP (podocyte EVs), mTomato (all other EVs), and positive for both signals were set. mGFP⁺ population or mTomato⁺ population were analyzed for rbIgG (Ch11) and Annexin V (Ch11) signal (mGFP⁺ population: Ch11 against Ch02). The same was done for the mTomato⁺ population (Ch03). For BALB/c mice MemBrite^{®} 594/615 positive EVs were used as total vesicle population. Ch04 MemBrite594/615 fluorescence plotted against SSC Ch06. All events that showed low SSC (<1000) but a fluorescent intensity for MemBrite594/615 were used as total EVs for further analysis. The MemBrite594/615⁺ population was plotted for the marker rbIgG (cy5 Ch11) and 14-3-3 (AF546 Ch03). Human EV gating: Ch04 MemBrite594/615 fluorescence plotted against SSC Ch06. All events that showed low SSC (<1000) but a fluorescent intensity for MemBrite594/615 were used as total EVs for further analysis. The MemBrite594⁺ population was plotted for the marker huIgG4 in combination with 14-3-3, Annexin A1, Annexin V, and CD63/81 with uniform gates.

Data analysis was performed using Amnis IDEAS software (version 6.2).

### Nano particle tracking (NTA) analysis

EV size distributions within EV fractions were assessed by NTA analyses. For NTA measurement of patient EV fractions (input, rest EVs, huIgG4⁺-EVs), 109 EVs per fraction were applied. For this purpose, huIgG4⁺-EVs were freed from huIgG4-beads via thorough vortexing for 3 min prior to quantification by image stream. For NTA analyses of human podocyte EV size distribution, 10⁵ EVs were used.

Briefly, EVs were diluted at 1:1000 in 1.5 ml PBS and 500 µL were loaded into the sample chamber of an LM10 unit (Nanosight, Amesbury, UK). Measurement settings were set to screen gain = 2 and camera level = 15. Five videos of one minute duration with 25°C temperature control inside the chamber were recorded for each sample. Data analysis was performed with NTA 3.0 software (Nanosight). Software settings for analysis were as follows: detection threshold = 6, screen gain = 10. The maximum particle size detected from the instrument as true particles was 5 µm.

### Immunoblot

Immunoblots were performed with human podocyte cell lysates, whole glomerular lysates, soluble versus insoluble mouse glomerular lysates, human podocyte EV lysates, and human patient urine EV lysates.

*Patient urinary huIgG4*⁺*-EVs*: HuIgG4⁺-EVs bound to the huIgG4-beads were separated from the beads by thorough vortexing for 3 min. Beads were removed by DynaMag and the supernatant containing the free huIgG4⁺-EVs was removed to determine the EV concentration via image stream. 104 enriched huIgG4⁺-EVs were lysed in 10 µl PBS with 2 µl 5x loading buffer [250 mM Tris-HCl pH 6.8, 10% SDS, 0,5 M DTT, 50% glycerol und 0.25% bromophenol blue], incubated for 10 min at RT and loaded on an SDS-PAGE and processed for immunoblotting as detailed below. Of note, EV protein abundances determined by immunoblotting are suitable for qualitative observations ("protein xy is present"). Quantitative observations ("protein xy is more abundant in sample A than in B") are limited due to normalization issues: 1) normalization to absolute EV number is problematic due to the size variations of EV populations, which leads to different total protein abundances; 2) normalization to protein content is problematic, as BCA measurements are not reliable from the nephrotic urine despite all attempts to reduce albumin contamination; 3) normalization to home keeper is hampered by the lack of adequate and uniform EV home keepers within different EV fractions.

*Human podocyte culture EVs:* Total EVs isolated from 3 ml exosome-depleted medium were resuspended in 12 µl PBS. Subsequently, 3 µl of a 5-fold loading buffer was added, samples were heated for 10 min at 95°C, cooled and completely loaded for SDS-PAGE analyses.

*Murine glomeruli:* For fractionation into a soluble and insoluble pellet, glomeruli were subsequentially lysed: First, the soluble fraction was obtained by adding 30 µl of T-Per containing 1 mM sodium fluoride (Sigma, #S1504), 1 mM sodium orthovanadate (Sigma Aldrich, #S6508), 100 nM Calyculin A (Sigma, #208851), 1x SigmaFast EDTA-free protease inhibitor cocktail (Sigma, #S8830) per 1000 glomeruli. Glomeruli were mechanically shredded with a pestle within the lysis buffer, incubated for 30 min on ice with vortexing every 5 minutes. Lysed glomeruli were centrifuged for 30 min, 4°C at 16000 x g and the supernatant containing the soluble proteins was collected. For the insoluble fraction, the remaining pellet was lysed in 30 µl per 1000 glomeruli urea buffer [8 M Urea, 10 mM DTT in 50 mM Tris, pH 8,0], shredded with a pestle, incubated for 30 min on ice with vortexing every 5 minutes, centrifuged for 30 min at 16000 x g, 4°C and the supernatant with insoluble proteins was collected. 6 µl of the soluble and insoluble fractions (according to 200 glomeruli) were then denatured with SDS solubilization buffer [250 mM Tris-HCl pH 6.8, 10% SDS, 0,5 M DTT, 50% Glycerol und 0.25% Bromphenolblue] and loaded on an SDS-PAGE.

In general, samples were separated on a 4-15% MiniProtean TGX gel (BioRad, #4568083, 4568085, or 4568086) in a Tris-glycine migration buffer (0.25 M Tris, 1.92 M glycine, 1% SDS, pH 8.3). Protein transfer was performed in transfer buffer (0.192 M glycine, 25 mM Tris base, 20% EtOH in H2O) in a TransBlot Turbo System (BioRad). After the transfer, all proteins were visualized by Ponceau staining. PVDF membranes (Merck-Millipore, #IPVH00010) were blocked (3% nonfat milk in TBS-T [10 mM Tris pH 7.4, 100 mM NaCl, 0.05% Tween20; all ThGeyer, #11784154, #11646918, #116597745]) prior to incubation with primary antibodies diluted in Superblock blocking reagent (Thermo Fisher, #37535) or non-fat milk in TBS-T. Binding was detected by incubation with HRP-coupled secondary antibodies (1:10.000, 3% nonfat milk in TBS-T). Before and after incubation of the membrane in secondary antibody, the membrane was washed three times for 10 minutes in TBS-T. Protein expression was visualized with Chemiluminescent Substrate West Pico Plus (Thermo Fisher, #34578) or Chemiluminescent Substrate Femto Super Signal Maximum Sensitivity Substrate (Thermo Fisher, #34095) according to manufacturer's instructions on Amersham ImageQuant 800 (Cytiva). Western blots were analyzed using software from ImageJ ([1]). Normalization was performed to either ponceau, β-actin, or vesicular markers stained on the same membrane for densitometric quantification. If experiments from different membranes were pooled, an internal calculation of relative changes to control within one membrane was assessed. Bands of the same membrane are shown, fine dashed white lines indicate, where bands were not adjacent to another on the membrane.

### Autoantibody elution from urinary patient vesicles

Autoantibodies were eluted from the HuIgG4-bead-bound EVs (= sample 2, Fig. 7) as follows. Beads with bound EVs were incubated in 50 µl 0.1M glycine pH 3.5 for 3 min and vortexed vigorously for 1 min. Similar volume of neutralization buffer (0.1M Tris pH 8) was added. The first supernatant containing the EVs and autoantibodies was collected on a DynaMag. To elute remaining EVs and autoantibodies, beads were reincubated with 0.1M glycine pH 3.5, vortexed and neutralized. The second supernatant was pooled with the first one. Beads were removed by DynaMag and the EV concentration was measured by image stream. To remove the EVs from the supernatant, the supernatant was ultra-centrifuged for 1.5 hours, 100.000 x g at 4°C. The supernatant containing the eluted autoantibodies (= sample 4, Fig. 7) was stored for subsequent use as primary immunoblot antibody.

### Native proteasome activity

Native proteasome activity assay was performed with human podocyte cell and EV lysates. 2.4 x 10⁵ cells and 1.3 × 10⁶ EVs (as determined by image stream) were lysed in TSDG buffer [10 mM Tris, 10 mM NaCl, 25 mM KCl, 1 mM MgCl₂, 0.1 mM EDTA, 10 % glycerol, 1mM DTT, 2 mM ATP, pH 7.5]. Cells were lysed in 40 µl TSDG buffer, EVs in a total volume of 11.52 µl TSDG buffer by performing 7 freeze and thaw cycles on a mixture of pure ethanol and dry ice and a 42°C water bath. Cell lysates were centrifuged for 10 minutes at 16000 x g at 4°C. Cell lysate supernatant (5 µg) and total EV lysates were used for proteasome activity measurements with the pan-proteasomal activity-based probe (ABP) cy5-epoxomicin (kind gift from B. Florea, Bio-organic synthesis group, Leiden University, Leiden, the Netherlands). Lysates were incubated with 4 µM cy5-epoxomicin for 1h at 37°C in a total volume of 12 µl. Samples were mixed with 5 x native sample buffer [250 mM BisTris, 250 mM NaCl, 50% glycerol, pH 6.5] and directly loaded to a 3-12% native gel (Serva, #43250). Proteins were separated for 3 hours at 150 V in native running buffer [50 mM BisTris, 50 mM Tricin, 0.4 mM ATP, 2 mM MgCl2, 0.5 mM DTT]. Protein transfer and immunoblot were performed as described [57]. 20S Proteasome loading was assessed by blotting for the a3-subunit, which is present in all proteasome constitutions.

### Proteomics of HuIgG4⁺-EVs

The huIgG4-bead-bound EVs (= sample 2, Fig. 7) were washed and freed from the beads as follows. 50 µl 0.15M sodium carbonate pH11 was added, incubated for 1 min, vortexed thoroughly for 2 minutes and neutralized with similar volume of 0.1M Tris pH8.0. On a DynaMag the supernatant containing the huIgG4⁺-EVs was removed, and the beads were incubated again with 0.15 M sodium carbonate pH 11.0, vortexed and neutralized to free remaining EVs from Beads. Beads were removed by DynaMag and EV concentration was measured by image stream. HuIgG4⁺-EVs were ultra-centrifuged for 1.5 hours at 100.000 x g, 4°C. Supernatant was discarded and EVs were prepared for proteomics. To do so, EVs were lysed in 20 mM HEPES, pH8, with 1% (v/v) sodium dodecyl sulfate (SDS) followed by 1 min incubation at 95°C. Extraction was enhanced by one freezing step in liquid nitrogen and thawing at room temperature while shaking for 15 min at 1400 rpm (Eppendorf). After a 5 min incubation of samples in an ultrasonication bath, samples were centrifuged for 60 min at 17,000 x g and room temperature. The supernatant containing soluble proteins was subjected to protein concentration determination using a BCA assay with bovine serum albumin as standard (Micro BCA Protein Assay Kit, Thermo Fisher Scientific).

Each 1.5 µl sample referring to 3*10⁵ EVs, were supplemented with 550 ng 15N-labelled protein extract of Bacillus subtilis as normalization standard and filled up with HEPES 20 mM, pH 8 to 10 µl. Then, disulfide bounds were reduced for 30 min with dithiothreitol (2.5 mM final concentration) and free SH-groups at cysteine were subsequently alkylated using iodoacetamide (10 mM final concentration) for further 30 min in the dark. Then, 8 µl of 20 µg/µl SeraMag bead mix were added to each sample in order to prepare protein extracts for tryptic digestion (protein : trypsin ratio 25 : 1) using the SP3 bead-based protocol [10]. After stopping the trypsin reaction with 0.5% (v/v) trifluoric acid and separation peptides from the beads using a magnetic rack and centrifugation, peptides were investigated by nanoLC-MS/MS using an Orbitrap ExplorisTM mass spectrometer (Thermo Fisher Scientific) coupled to an Ultimate 3000 RSLC (Thermo Fisher Scientific). Peptide separation of 1 µl peptide solution (referring to peptides of 1.23*10⁴ EVs) was accomplished with a solvent gradient from 0 to 100% acetonitrile in 0.1% acetic acid and mass spectrometry data were acquired in data independent mode.

Data analysis was accomplished using Spectronaut 18.6 (Biognosys). First, a spectral library was created from a sample set containing the mentioned fractions of exophers of the four urine samples by comparing against a Uniprot database limited to human entries (2022). The following parameter settings were employed: tryptic digestion, up to two missed-cleavages allowed, carbamidomethylation at cysteine as fixed and oxidation at methionine as variable condition. The library was then used to compare spectra with each pellet and supernatant fraction per urine sample. In addition, an existing B. subtilis 15N spectral library was enabled. Detailed search settings are provided as Suppl. Table 4.

Statistical analysis was performed using R version 4.3.0. The Spectronaut ion report was loaded into R for further processing. The ions were median normalized with respect to the sample-specific B. subtilis spike-in signal per sample. Only the normalized ions with a Q-value below 0.001 were utilized for the iBAQ protein intensity calculation. For this calculation, only proteins with 2 or more peptides identified per sample with a Q-value below 0.001 were considered. The resulting iBAQ values were then filtered to retain only unique protein groups.

The final list of proteins per sample, derived from this process, was used for the gProfiler analysis. This analysis was conducted using the gprofiler2 [39] package (v0.2.2) using g:SCS multiple testing correction method the significance threshold was set to 0.05. Plots were generated using tidyverse (v2.0.0), ggtext (v0.1.2), glue (v1.7.0), ggh4x (v0.2.6) packages.

### Statistical analysis

Results were expressed as mean ± SEM, and significance was set at *p < 0.05. The means in a time course were compared using the two-way ANOVA using Prism 8.2.1 for Mac OS X, GraphPad Software, San Diego, California USA, www.graphpad.com. Animal experiments were compared using the one-way ANOVA Kruskal-Wallis test followed by the Dunn's post-test for multiple comparisons using Prism 8.2.1 for Mac OS X, GraphPad Software, San Diego, California USA, www.graphpad.com. Animal experiments with only two groups were compared using the Mann-Whitney U test. Replicates used were biological replicates, which were measured using different samples from distinct experiments. All mice were littermates and were blindly assigned to the experimental groups.

### Results

### Exophers are the pathobiological correlate of glomerular urinary space aggregates in MN.

The analyses of diagnostic biopsies and diagnostic/consecutive urine samples from a THSD7A⁺-MN patient and from three PLA₂R1⁺-MN patients revealed human exopher-formation as an antigen-overarching principle in MN, compared to nephrotic non-MN patients (two minimal change disease, two primary FSGS, one tubulo-toxic kidney injury, and one IgA nephritis patient). In the diagnostic biopsies of MN patients, glomerular antigen aggregates were identified within the urinary space that had a membrane connection to podocytes (Fig. 1A, panel 1. Fig. 2A). After urinary release, antigen aggregates were found in adherence to the tubular brush border (Fig. 1A panel 2, Fig. 2B), reminiscent of the 'starry night' phenotype of burst *C*. *elegans* exophers [46]. Additionally, antigen aggregates localized to the cytoplasm of parietal epithelial and tubular cells, demonstrating cellular reuptake of aggregates released from the glomerulus (Fig. 1A panel 3, Figs. 2C). Antigen aggregates were strongly positive for huIgG4 identifying them as immune complexes within the glomerular urinary space and tubular lumen (Fig. 1B and Fig. 2D panels 1). Strikingly, antigens colocalized stronger with huIgG4 within urinary space aggregates than within the subepithelial space deposits, as observed by confocal microscopy. Demonstrating their urinary release, a subset of large (~5-8 µm) extracellular vesicles (EVs) was discerned among urinary EVs of MN patients, in which the MN antigen and huIgG4 strongly colocalized. These EVs also showed a granular positivity for the EV marker 14-3-3 (Fig. 1B and Fig. 2D, panels 2). The mean size of huIgG4⁺-EVs ranged from ~580 nm in the THSD7A⁺-MN patient and ~450 nm in a PLA₂R1⁺-MN patient (Fig. 2E panel 1). The EV-bound huIgG4 antibodies exhibited a specific reactivity to human THSD7A or PLA₂R1 protein upon elution, establishing them as the pathogenic MN autoantibodies (Fig. 1B and Fig. 2D panels 3). As shown by immunofluorescence, immunoblotting, and image stream analyses, huIgG4⁺-EVs expressed proteins such as 14-3-3, Annexin A1, CD63/CD81 indicating their vesicular origin (Fig. 1C, Fig. 2E). To this end, 14-3-3 represented the most discerning marker in huIgG4⁺-EVs and was recently shown to be required for exopher-formation in *C*. *elegans* neurons (5). In comparison to nephrotic non-MN patients, huIgG4⁺-EVs isolated from MN patient urines contained abundant podocyte proteins such as THSD7A, PLA₂R1, Nephrin, Podocin, and α-Actinin4 by immunoblot or by immunofluorescence (Fig. 1D and Fig. 2F) showing their podocyte origin. Besides podocyte proteins, disease-associated proteins such as complement (C1q, C5b9), mitochondrial (Mitofusin 2, ATPB), lysosomal (LIMP2, LAMP2), and ubiquitin proteasome system (UPS) proteins (K48-polyubiquitinated proteins as proteasome substrates, UCH-L1 and the immunoproteasome subunit LMP7) were abundant by immunoblot or by immunofluorescence in huIgG4⁺-EVs from MN patient urines (Fig. 1E, Fig. 3). Showing the glomerular origin of these huIgG4⁺-EVs, confocal analyses within the diagnostic biopsies of THSD7A⁺- and PLA₂R1⁺-MN patients localized selected disease-associated proteins to huIgG4⁺ aggregates within the glomerular urinary space (not shown) and within the tubular lumen (not shown). Together, these analyses show the formation of exophers in MN glomeruli that contain the MN antigen with bound autoantibody, 14-3-3, and disease-associated proteins, which are released by the podocyte and passage through the nephron for cellular uptake or urinary removal.

The urinary exophers identified in MN patients were unique in respect to their appearance and content compared to urinary huIgG4⁺-EVs from nephrotic non-MN patients as summarized in Fig. 4. HuIgG4⁺-EVs from nephrotic non-MN patients were mostly low abundant or devoid of podocyte proteins, complement, proteasome substrates, mitochondria, and lysosomes, among others (Fig 1, Fig. 2). Further, they were smaller and had a less aggregate-like huIgG4 expression pattern by immunofluorescence compared to MN exophers (not shown). Interestingly, the urinary abundance of huIgG4⁺/14-3-3⁺-EVs strongly differed between patient groups. Primary FSGS and tubulo-toxic kidney injury patients exhibited a low abundance (-0.6-1.2%), whereas MCD and IgAN patients exhibited a high abundance of huIgG4⁺/14-3-3⁺-EVs ranging from ~25% in MCD and ~10% in the IgAN patient, irrespectively of the extent of proteinuria (not shown). No specific reactivity to glomerular/podocyte/HEK cell proteins could be identified from the immunoglobulins eluted from the isolated huIgG4⁺-EVs of non-MN patients (not shown).

In summary, these findings identify the glomerular urinary space aggregates in MN as exophers, which are accessible to non-invasive molecular analyses through their urinary release as huIgG4⁺/14-3-3⁺-EVs.

### Non-invasive exopher monitoring in relapsing THSD7A⁺-MN.

Urinary exopher-release and as such the podocytes' ability to deal with circulating autoantibodies was prospectively monitored for 2½ years in a 62-year-old male who was diagnosed with THSD7A⁺-MN in May 2021. The patient initially presented with heavy nephrotic syndrome and normal renal function. His diagnostic histopathology was significant for a THSD7A⁺-MN (stage I Ehrenreich and Churg), with segmental thickened GBM (Fig. 5A panel 1), a pseudolinear positivity for huIgG (Fig. 5A panel 2), a fine glomerular positivity for huIgG4 at the filtration barrier (Fig. 5A panel 3), and THSD7A aggregates. THSD7A aggregates were discreet in the subepithelial space and prominent in the urinary space, some within parietal epithelial cells (Fig. 5A panel 4). Ultra-structurally, foot process effacement and GBM thickening were seen, with some long membrane extensions derived from foot and major processes (Fig. 5A panel 5). The clinical course of the patient (Fig. 5B graph) showed a relapsing nephrotic syndrome complicated by cerebral infarctions (09/2021 and 10/2022), for a progressive decline of renal function (eGFR 90 ml/min at diagnosis to 33 ml/min 12/2023). The patient received several courses of rituximab as well as a cyclophosphamide pulse therapy, under which he developed a symptomatic COVID-19 infection. Besides the diagnostic urine (U1), 7 additional urines (U2-U8) were collected and analyzed for exopher abundance (Fig. 5B, panels 1) and for the presence of exopher-bound autoantibodies (Fig. 5B, panels 2). Albeit the presence of high sTHSD7A-ab titers of 1:160, urinary exopher release progressively decreased from ~35% at diagnosis to ~3% in U3. From 10/2022 on, sTHSD7A-abs were mostly negative, nonetheless autoantibodies were persistently eluted from urinary exophers. Four months after the last rituximab administration, the patient presented with increasing edemas and proteinuria (28/06/2023). Upon immunological checkup, a low but recovering CD19⁺ B-cell count was noted (2%; 14/µl), sTHSD7A-abs were still not detectable. Nonetheless, analyses of U7 showed an increase in urinary exopher abundance (4%) which contained bound autoantibodies demonstrating immunologic activity. Rituximab administration ameliorated the patients' proteinuria and edema. U8 collected 4½ months later still in the setting of negative sTHSD7A-abs, exhibited a low exopher abundance (-0.2%) with readily detectable exopher-bound autoantibodies.

The patients' exophers enriched from U1-U8 were used for proteomic molecular disease patterning (Fig. 5B, panel 3). These unbiased analyses not only substantiated the vesicular/plasma membrane origin of exophers, but also the enrichment of disease associated pathways such as cell stress response (heat shock proteins, chaperones), classical complement and humoral immune system, as well as proteolytic pathways (proteasome and lysosome) established within our biased analyses. Even though a molecular imprint of the podocyte injury state was visible over all investigated time points, a certain dynamic in the proteome profile was present.

Glomerular aggregate morphometrics and non-invasive exopher monitoring in PLA₂R1⁺-MN.

Patients with THSD7A⁺-MN are rare, therefore we developed a computational image algorithm to quantify the abundance of urinary-space PLA₂R1 aggregates as the biological correlate of podocyte exopher-formation. The `density score' of this algorithm included the size, amount, neighborhood, and intensity of the PLA₂R1 aggregates throughout the glomeruli. A low density score related to the predominance of urinary space aggregates and a disrupted subepithelial PLA₂R1 deposition pattern, whereas a high density score reflected the occurrence of dense subepithelial PLA₂R1 depositions and scarce urinary space aggregates. PLA₂R1 density differed in diagnostic biopsies of a well-characterized retrospective PLA₂R1⁺-MN patient cohort [57] in which 18 patients were stratified to a remission group (R; amelioration of serum creatinine, eGFR, proteinuria, albuminuria, and/or serum albumin) within a mean follow-up period of 42,83 ± 5,52 months, and 17 patients in a non-remission (NR) group, within a mean follow-up period of 43,24 ± 9,2 months. In the remission group, PLA₂R1 density and sPLA₂R1-abs were low at diagnosis. The non-remission group exhibited a high PLA₂R1 density as well as high sPLA₂R1 titers at diagnosis (not shown).

Exopher-genesis was prospectively analyzed in a 20-year-old female nephrotic patient diagnosed 01/2021 with PLA₂R1⁺-MN (stage I-III Ehrenreich and Churg) based on the clinical and nephropathological workup (patient P2). At diagnosis sPLA₂R1-ab titer was moderate (92 RE/ml). Urinary exopher diagnostics identified ~8% exophers, and exopher-bound PLA₂R1 autoantibodies were readily detectable (not shown). Morphologically, PLA₂R1 density was low in the diagnostic biopsy which related to a disrupted subepithelial PLA₂R1 deposition and small glomerular urinary-space PLA₂R1 aggregates (not shown). Together, these findings indicated a low glomerular aggregate burden in the setting of moderate sPLA₂R1-ab titer, with dynamic exopher-formation and release at diagnosis. Within one year the patient went into clinical remission under a supportive treatment regimen, and cyclosporin A. A relapse 01/06/2022 was treated with rituximab. Serum PLA₂R1 titers were first negative 10/08/2022 and remained negative thereafter, suggesting immunologic remission. In the last follow-up urine U2 (16/10/2023) exopher abundance was low (-0.7%) and no exopher-bound PLA₂R1 autoantibodies were detectable, substantiating immunologic remission.

Based on these patient observations we propose that simultaneously tracking the fate of pathogenic autoantibodies (serum levels, glomerular binding, and release) enables a fine-tuned monitoring of immunologic MN disease activity, especially in the setting of negative serum autoantibody titers.

### Exophers form at podocyte processes and cell body in experimental THSD7A⁺-MN.

Experimental MN was induced in mice by intravenous injection of rbTHSD7A-abs [78]. Scanning electron microscopy of day 1, 3, and 7 kidneys revealed that podocytes formed long membrane extensions with distal bulges that extended into the urinary space in response to rbTHSD7A-ab exposure. These structures were reminiscent of exophers by morphology [46] and increased in length and abundance over time. The membrane extensions originated from foot and major processes as well as from the cell body. Additionally, large protrusions with rough surfaces without a stalk were focally observed at the urinary side of podocyte processes and cell body. The immunogold detection of the bound rbTHSD7A-abs on day 7 abundantly localized rbIgG to the distal ends of the long membrane extensions as large, gold-labeled mulberry-like structures within the glomerular urinary space by transmission EM. Additionally, extensive gold-labeled aggregates were observed that directly protruded from the urinary side plasma membranes of podocyte foot and major processes. The 'expected' subepithelial deposition of rbTHSD7A-abs was discreet on day 7. In contrast, subepithelial deposition of rbTHSD7A-abs was abundant on day 1, whereas gold-labeling of podocyte processes within the glomerular urinary space was scarce. No intracellular gold-particles were observed at the evaluated time points. Immunogold labeling of THSD7A as the corresponding antigen also discerned gold-labeled THSD7A within and at the distal end of podocyte-derived membrane extensions that reached into the urinary space. Further, gold-labeled THSD7A was present within aggregates protruding from podocyte foot processes. This pattern corresponded to the rbIgG immunogold aggregate pattern described above.

Like in MN patients, THSD7A⁺-MN mice released glomerular urinary space aggregates to the urine. Vesicular structures with gold-labeled rbTHSD7A-abs were observed contacting proximal tubular brush borders. By confocal microscopy THSD7A⁺/rbIgG⁺ complexes were seen within proximal tubular and parietal epithelial cells showing their reuptake. Within the urine of THSD7A⁺-MN mice, a subset of released EVs were exophers, identified as large rbIgG⁺/14-3-3⁺ EVs, with a mean size of ~470, and a maximum size of ~5 µm.

### Exopher-formation encompasses antigen crosslinking, translocation, and release in podocytes.

Human podocytes that overexpress THSD7A in culture are specifically targeted by patient [77] and rabbit-THSD7A-antibodies [78] in vitro. Therefore, they are ideal to investigate the podocytes' response to MN autoantibody (ab) binding. Morphologically, in vitro exposure of podocytes to human (hu)THSD7A-abs rapidly induced the formation of honeycomb-like autoantibody patches at the luminal cell surface, which predominated at the cell periphery, indicating antigen crosslinking by the autoantibody. Live-cell imaging discerned areas of plasma membrane stiffening at the cell periphery, which were the starting point of exopher-formation after exposure to rbTHSD7A-abs. Translocation of peripheral stiffened plasma membrane towards juxtanuclear cell areas could be observed, followed by a release as stalked, large EVs to the medium. The abluminal side of podocytes showed no alterations in holotomographic and confocal 3D reconstructions. EVs protruding from the luminal side of podocytes had a high refractive index as observed using holotomography. Confocal resolution demonstrated that rbIgG localized to the outer aspect of THSD7A within EVs that protruded at the juxtanuclear plasma membrane area. Further, the EV markers Annexin A1 and 14-3-3 were highly abundant in protruding EVs, 14-3-3 in an aggregate pattern. Together, this identifies the protrusions as forming exophers.

The majority of exophers were released from podocytes within 6 hours of rbTHSD7A-ab exposure and were rbIgG⁺ by image stream. Released exophers were large (mean ~470 nm, max ~4,5 µm) by NTA measurement. Molecular analyses demonstrated that already within 1 hour of in vitro exposure to rbTHSD7A-abs, podocytes released substantial amounts of THSD7A within exophers to the medium, which matched the loss of THSD7A within the corresponding podocyte lysates. Exophers did not only reduce the podocyte membrane burden of THSD7A and bound autoantibody but also substantiated a cellular exit route for disease-associated proteotoxic material. As such, they were abundant of 20S proteasome complexes with impaired proteolytic activity. Additionally, selected exophers exhibited an uptake of Lysotracker and MitoSOX dyes, demonstrating the release of lysosomes and damaged mitochondria in exophers, respectively. Together these data show that autoantibody binding to THSD7A initiates crosslinking of the antigen at the podocytes' peripheral membranes. Stiffened plasma membrane translocates to juxtanuclear membrane areas to be released as stalked, large exophers. As part of the proteotoxic disease process, exophers contain THSD7A with the bound autoantibody, lysosomes, dysfunctional proteasomes, and damaged mitochondria.

### Podocyte exopher-release is protective in experimental THSD7A⁺-MN.

Exopher-formation and urinary release can be regarded as a beneficial reaction in MN. Image stream analyses using mT/mG reporter mice allowed differential tracking of podocyte-derived (GFP⁺) EVs and non-podocyte (tdTomato⁺) EVs. Following exposure to rbTHSD7A-abs but not to ctrl-abs, podocyte-derived EVs were increasingly released. A subfraction of podocyte-derived EVs was rbIgG⁺ and negative for the apoptotic body marker Annexin V, as described for exophers

[46]. The release of non-podocyte-EVs was not affected, demonstrating the specificity of the exopher-release from podocytes in experimental MN. With beginning albuminuria 1 day after THSD7A⁺-MN induction, urinary release of exophers was discernible, which peaked on day 3. Thereafter, exopher-release decreased, whereas albuminuria continued to worsen (Fig. 6A, panel 1). Acceleration of exopher-release through short-term proteasome inhibition starting day 7 momentarily decreased albuminuria compared to vehicle treated THSD7A⁺-MN mice (Fig. 6A panel 2). Exophers were the only urinary EVs whose release was affected by proteotoxic stress. At the end of the observation period, the extent of urinary exopher-release inversely correlated with albuminuria, as well as with the glomerular deposition of rbIgG or THSD7A protein in experimental mice (Fig. 6A panel 3), demonstrating that exopher-release is protective in MN.

'Long-term' proteasome inhibition beginning prior to THSD7A⁺-MN induction exacerbated urinary exopher-release especially on day 7 relative to vehicle treated mice (Fig. 6B, panel 1). Urinary exopher-release, however, diminished with prolonged proteotoxic stress relative to vehicle treated mice, despite the persistence of circulating autoantibodies in the serum. The decrease in exopher-release was mirrored by an exacerbation of albuminuria. Morphologically, proteasome-inhibited THSD7A⁺-MN mice displayed larger glomerular aggregates in the urinary space than vehicle treated THSD7A⁺-MN mice. Identifying them as exophers, these rbIgG⁺ aggregates were in connection with the podocyte cell body through long mGFP⁺ membrane extensions in mT/mG mice. Interestingly, glomerular exophers of proteasome-inhibited mice were large, especially in the setting of prolonged proteotoxic stress (Fig. 6B panel 2), suggesting that the decrease in urinary exopher abundance at the end of the observation time resulted from impaired release rather than formation.

As summarized in Fig 6C, these investigations demonstrate that podocyte exopher-formation and release represent a protective antigen-overarching pathophysiologic sequel in MN. We propose that simultaneously tracking the fate of pathogenic autoantibodies (serum levels, glomerular binding, and release, Fig. 6C, panel 1) enables fine-tuned monitoring of immunologic MN disease activity. Mechanistically, exopher-formation includes the displacement of antigen/autoantibodies from the subepithelial space to the apical membranes of foot and major processes and cell body (Fig. 6C, panel 2). Exopher-release to the urine ultimately leads to a reduction of the glomerular subepithelial autoantibody and antigen load (Fig. 6C, panel 3).

### References

1 Abràmoff MD, Magalhães PJ, Ram SJJBi. Image processing with ImageJ. 2004;11(7):36-42
2 Alok A, Yadav A. Membranous Nephropathy. [Updated 2023 Jun 5]. In: StatPearls [Internet]. Treasure Island (FL): StatPearls Publishing; 2024 Jan-. Available from: https://www.ncbi.nlm.nih.gov/books/NBK559169/
3 Augert A, Vindrieux D, Girard CA, et al. PLA2R1 kills cancer cells by inducing mitochondrial stress. Free Radic Biol Med 2013;65:969-977. DOI: 10.1016/j.freeradbiomed.2013.08.177.
4 Arnold ML, Cooper J, Grant BD, Driscoll M. Quantitative Approaches for Scoring in vivo Neuronal Aggregate and Organelle Extrusion in Large Exopher Vesicles in C. elegans. J Vis Exp 2020(163). DOI: 10.3791/61368;
5 Arnold ML, Cooper J, Androwski R, et al. Intermediate filaments associate with aggresome-like structures in proteostressed C. elegans neurons and influence large vesicle extrusions as exophers. Nat Commun 2023;14(1):4450. DOI: 10.1038/s41467-023-39700-1;
6 Banasiak K, Turek M, Pokrzywa W. Preparation of Caenorhabditis elegans for Scoring of Muscle-derived Exophers. Bio Protoc 2023;13(1). DOI: 10.21769/BioProtoc.4586.
7 Beck LH, Jr., Bonegio RG, Lambeau G, et al. M-type phospholipase A2 receptor as target antigen in idiopathic membranous nephropathy. N Engl J Med 2009;361(1):11-21. DOI: 10.1056/NEJMoa0810457;
8 Beck LH, Jr. PLA2R and THSD7A: Disparate Paths to the Same Disease? J Am Soc Nephrol 2017;28(9):2579-2589. DOI: 10.1681/ASN.2017020178.
9 Bikbov, Boris et al. 2020, Global, regional, and national burden of chronic kidney disease, 1990-2017: a systematic analysis for the Global Burden of Disease Study 2017, The Lancet 395, 709 -733, DOI: 10.1016/S0140-6736(20)30045-3;
10 Blankenburg S, Hentschker C, Nagel A, et al. Improving Proteome Coverage for Small Sample Amounts: An Advanced Method for Proteomics Approaches with Low Bacterial Cell Numbers. Proteomics 2019;19(23):e1900192. DOI: 10.1002/pmic.201900192.
11 Boerries M, Grahammer F, Eiselein S, et al. Molecular fingerprinting of the podocyte reveals novel gene and protein regulatory networks. Kidney Int 2013;83(6):1052-64. DOI: 10.1038/ki.2012.487.
12 Boersma HH, Kietselaer BL, Stolk LM, Bennaghmouch A, Hofstra L, Narula J, Heidendal GA, Reutelingsperger CP. Past, present, and future of annexin A5: from protein discovery to clinical applications. J Nucl Med. 2005 Dec;46(12):2035-50.
13 Buzas EI. The roles of extracellular vesicles in the immune system. Nat Rev Immunol. 2023 Apr;23(4):236-250. doi: 10.1038/s41577-022-00763-8.
14 Chen TK, Knicely DH, Grams ME. Chronic Kidney Disease Diagnosis and Management: A Review. JAMA. 2019 Oct 1;322(13):1294-1304. doi: 10.1001/jama.2019.14745.
15 Cooper JF, Guasp RJ, Arnold ML, Grant BD, Driscoll M. Stress increases in exopher-mediated neuronal extrusion require lipid biosynthesis, FGF, and EGF RAS/MAPK signaling. Proc Natl Acad Sci U S A 2021;118(36). DOI: 10.1073/pnas.2101410118;
16 Davis AA, Leyns CEG, Holtzman DM. Intercellular Spread of Protein Aggregates in Neurodegenerative Disease. Annu Rev Cell Dev Biol 2018;34:545-568. DOI: 10.1146/annurev-cellbio-100617-062636.
17 Donnelly KM, DeLorenzo OR, Zaya AD, et al. Phagocytic glia are obligatory intermediates in transmission of mutant huntingtin aggregates across neuronal synapses. Elife 2020;9. DOI: 10.7554/eLife.58499.
18 Doyle LM, Wang MZ. Overview of Extracellular Vesicles, Their Origin, Composition, Purpose, and Methods for Exosome Isolation and Analysis. Cells. 2019 Jul 15;8(7):727. doi: 10.3390/cells8070727.
19 Ershov D, Phan MS, Pylvanainen JW, et al. TrackMate 7: integrating state-of-the-art segmentation algorithms into tracking pipelines. Nat Methods 2022;19(7):829-832. DOI: 10.1038/s41592-022-01507-1.
20 Evangelisti A, Butler H, del Monte F, The Heart of the Alzheimer's: A Mindful View of Heart Disease, Frontiers in Physiology 11: 625974, 2021, DOI: 10.3389/fphys.2020.625974
21 Evans, M., Lewis, R.D., Morgan, A.R. et al. A Narrative Review of Chronic Kidney Disease in Clinical Practice: Current Challenges and Future Perspectives. Adv Ther 39, 33-43 (2022). https://doi.org/10.1007/s12325-021-01927-z
22 Falchi AM, Sogos V, Saba F, Piras M, Congiu T, Piludu M. Astrocytes shed large membrane vesicles that contain mitochondria, lipid droplets and ATP. Histochem Cell Biol 2013;139(2):221-31. DOI: 10.1007/s00418-012-1045-x;
23 Faul C, Donnelly M, Merscher-Gomez S, et al. The actin cytoskeleton of kidney podocytes is a direct target of the antiproteinuric effect of cyclosporine A. Nat Med 2008;14(9):931-8. DOI: 10.1038/nm.1857.
24 Feng, D., DuMontier, C. & Pollak, M.R. The role of alpha-actinin-4 in human kidney disease. Cell Biosci 5, 44 (2015). https://doi.org/10.1186/s13578-015-0036-8
25 Fogo AB, Lusco MA, Najafian B, Alpers CE. AJKD Atlas of Renal Pathology: Membranous Nephropathy. Am J Kidney Dis 2015;66(3):e15-7. DOI: 10.1053/j.ajkd.2015.07.006.
26 Fu H, Subramanian RR, Masters SC. 14-3-3 proteins: structure, function, and regulation. Annu Rev Pharmacol Toxicol 2000;40:617-47. DOI: 10.1146/annurev.pharmtox.40.1.617.
27 Gaffling S, Daum V, Steidl S, Maier A, Kostler H, Hornegger J. A Gauss-Seidel iteration scheme for reference-free 3-D histological image reconstruction. IEEE Trans Med Imaging 2015;34(2):514-30. DOI: 10.1109/TMI.2014.2361784.
28 Götz J, Halliday G, Nisbet RM, Molecular Pathogenesis of the Tauopathies Annual Review of Pathology: Mechanisms of Disease 2019 14:1, 239-261, DOI: 10.1146/annurev-pathmechdis-012418-012936;
29 Hadizadeh N, Bagheri D, Shamsara M, Hamblin MR, Farmany A, Xu M, Liang Z, Razi F and Hashemi E (2022) Extracellular vesicles biogenesis, isolation, manipulation and genetic engineering for potential in vitro and in vivo therapeutics: An overview. Front. Bioeng. Biotechnol. 10:1019821. doi: 10.3389/fbioe.2022.1019821
30 Haileamlak A. Chronic Kidney Disease is on the Rise. Ethiop J Health Sci. 2018 Nov;28(6):681-682. doi: 10.4314/ejhs.v28i6.1;
31 Hara M, Yanagihara T, Kihara I, Higashi K, Fujimoto K, Kajita T. Apical cell membranes are shed into urine from injured podocytes: a novel phenomenon of podocyte injury. J Am Soc Nephrol 2005;16(2):408-16. DOI: 10.1681/ASN.2004070564.
32 Herwig J, Skuza S, Sachs W, et al. Thrombospondin Type 1 Domain-Containing 7A Localizes to the Slit Diaphragm and Stabilizes Membrane Dynamics of Fully Differentiated Podocytes. J Am Soc Nephrol 2019;30(5):824-839. DOI: 10.1681/ASN.2018090941.
33 Hoxha E, Wiech T, Stahl PR, et al. A Mechanism for Cancer-Associated Membranous Nephropathy. N Engl J Med 2016;374(20):1995-6. DOI: 10.1056/NEJMc1511702.
34 Hoxha E, Reinhard L, Stahl RAK. Membranous nephropathy: new pathogenic mechanisms and their clinical implications. Nat Rev Nephrol 2022;18(7):466-478. DOI: 10.1038/s41581-022-00564-1.
35 Huang Y, Yu M, Zheng J. Proximal tubules eliminate endocytosed gold nanoparticles through an organelle-extrusion-mediated self-renewal mechanism. Nat Nanotechnol 2023;18(6):637-646. DOI: 10.1038/s41565-023-01366-7;
36 Jeppesen DK, Zhang Q, Franklin JL, Coffey RJ, Extracellular vesicles and nanoparticles: emerging complexities, Trends in Cell Biology 33, 2023, 667-681, DOI: 10.1016/j.tcb.2023.01.002
37 Kelly L, McGrath S, Rodgers L, McCall K, Tulunay Virlan A, Dempsey F, Crichton S, Goodyear CS, Annexin-A1: The culprit or the solution? Immunology. 2022; 166: 2-16. DOI: 10.1111/imm. 13455
38 Kerjaschki D, Schulze M, Binder S, et al. Transcellular transport and membrane insertion of the C5b-9 membrane attack complex of complement by glomerular epithelial cells in experimental membranous nephropathy. J Immunol 1989;143(2):546-52.
39 Kolberg L, Raudvere U, Kuzmin I, Vilo J, Peterson H. gprofiler2 -- an R package for gene list functional enrichment analysis and namespace conversion toolset g:Profiler. F1000Res 2020;9. DOI: 10.12688/f1000research.24956.2.
40 Kovesdy CP. Epidemiology of chronic kidney disease: an update 2022. Kidney Int Suppl (2011). 2022 Apr;12(1):7-11. DOI: 10.1016/j.kisu.2021.11.003;
41 Kravets I, Mallipattu SK, The Role of Podocytes and Podocyte-Associated Biomarkers in Diagnosis and Treatment of Diabetic Kidney Disease, Journal of the Endocrine Society, Volume 4, Issue 4, April 2020, bvaa029, DOI: 10.1210/jendso/bvaa029;
42 Liangsupree T, Multia E, Riekkola ML, Modern isolation and separation techniques for extracellular vesicles, Journal of Chromatography A 1636, 2021, 461773, DOI: 10.1016/j.chroma.2020.461773;
43 Liao P-S, Chen, T-S., Chen and Chung, P-C. A Fast Algorithm for Multilevel Thresholding. Journal of Information Science and Engineering 2001;17(5):713-727. DOI: 10.6688/JISE.2001.17.5.1.
44 Liu, YJ., Wang, C. A review of the regulatory mechanisms of extracellular vesicles-mediated intercellular communication. Cell Commun Signal 21, 77 (2023). DOI: 10.1186/s12964-023-01103-6
45 Liu Y, Li S, Rong W, et al. Podocyte-Released Migrasomes in Urine Serve as an Indicator for Early Podocyte Injury. Kidney Dis (Basel) 2020;6(6):422-433. DOI: 10.1159/000511504.
46 Melentijevic I, Toth ML, Arnold ML, et al. C. elegans neurons jettison protein aggregates and mitochondria under neurotoxic stress. Nature 2017, 542(7641):367-371. DOI: 10.1038/nature21362;
47 Meyer TN, Schwesinger C, Wahlefeld J, et al. A new mouse model of immune-mediated podocyte injury. Kidney Int 2007;72(7):841-52. DOI: 10.1038/sj.ki.5002450.
48 Meyer-Schwesinger C, Lambeau G, Stahl RA. Thrombospondin type-1 domain-containing 7A in idiopathic membranous nephropathy. N Engl J Med 2015;372(11):1074-5. DOI: 10.1 056/NEJMc 1500 130.
49 Nicolas-Avila JA, Lechuga-Vieco AV, Esteban-Martinez L, et al. A Network of Macrophages Supports Mitochondrial Homeostasis in the Heart. Cell 2020;183(1):94-109 e23. DOI: 10.1016/j.cell.2020.08.031;
50 Neubert P, Protzel P, Compact Watershed and Preemptive SLIC: On Improving Trade-offs of Superpixel Segmentation Algorithms. ICPR 2014:996-1001. DOI: 10.1109/ICPR.2014.181
51 Nicolas-Avila JA, Sanchez-Diaz M, Hidalgo A. Isolation of exophers from cardiomyocyte-reporter mouse strains by fluorescence-activated cell sorting. STAR Protoc 2021;2(1): 100286. DOI: 10.1016/j.xpro.2020.100286;
52 Nicolas-Avila JA, Pena-Couso L, Munoz-Canoves P, Hidalgo A. Macrophages, Metabolism and Heterophagy in the Heart. Circ Res 2022;130(3):418-431. DOI: 10.1161/CIRCRESAHA.121.319812;
53 Papendorf JJ, Krüger E, Ebstein F. Proteostasis Perturbations and Their Roles in Causing Sterile Inflammation and Autoinflammatory Diseases. Cells. 2022 Apr 22;11(9):1422. doi: 10.33 90/cells 11091422.
54 Rani S, Lai A, Nair S, Sharma S, Handberg A, Carrion F, Möller A, Salomon C, Extracellular vesicles as mediators of cell-cell communication in ovarian cancer and beyond - A lipids focus, Cytokine & Growth Factor Reviews 73, 2023, 52-68, DOI: 10.1016/j.cytogfr.2023.06.004.
55 Ray A, Speese SD, Logan MA. Glial Draper Rescues Abeta Toxicity in a Drosophila Model of Alzheimer's Disease. J Neurosci 2017;37(49):11881-11893. DOI: 10.1523/JNEUROSCI.0862-17.2017.
56 Rees P, Summers HD, Filby A, Carpenter AE, Doan M. Imaging flow cytometry: a primer. Nat Rev Methods Primers. 2022;2:86. doi: 10.1038/s43586-022-00167-x.
57 Reichelt J, Sachs W, Frombling S, et al. Non-functional ubiquitin C-terminal hydrolase L1 drives podocyte injury through impairing proteasomes in autoimmune glomerulonephritis. Nat Commun 2023;14(1):2114. DOI: 10.1038/s41467-023-37836-8;
58 Reinhard L, Wiech T, Reitmeier A, et al. Pathogenicity of Human anti-PLA2R1 Antibodies in Minipigs: A Pilot Study. J Am Soc Nephrol 2023. DOI: 10.1681/ASN.0000000000000068.
59 Rispens, T., Huijbers, M.G. The unique properties of IgG4 and its roles in health and disease. Nat Rev Immunol 23, 763-778 (2023). DOI: 10.1038/s41577-023-00871-z
60 Rojas-Rivera JE, Ortiz A, Fervenza FC, Novel Treatments Paradigms: Membranous Nephropathy, Kidney International Reports, Volume 8, Issue 3, 2023, 419-431, DOI: 10.1016/j.ekir.2022.12.011.
61 Ronco, P., Beck, L., Debiec, H. et al. Membranous nephropathy. Nat Rev Dis Primers 7, 69 (2021). https://doi.org/10.1038/s41572-021-00303-z.
62 Saez PJ, Barbier L, Attia R, Thiam HR, Piel M, Vargas P. Leukocyte Migration and Deformation in Collagen Gels and Microfabricated Constrictions. Methods Mol Biol 2018;1749:361-373. DOI: 10.1007/978-1-4939-7701-7_26.
63 Saleem MA, O'Hare MJ, Reiser J, et al. A conditionally immortalized human podocyte cell line demonstrating nephrin and podocin expression. J Am Soc Nephrol 2002;13(3):630-638. DOI: 10.1681/ASN.V133630.
64 Sandoz PA, Tremblay C, van der Goot FG, Frechin M. Image-based analysis of living mammalian cells using label-free 3D refractive index maps reveals new organelle dynamics and dry mass flux. PLoS Biol 2019;17(12):e3000553. DOI: 10.1371/journal.pbio.3000553.
65 Schiller B, He C, Salant DJ, Lim A, Alexander JJ, Quigg RJ. Inhibition of complement regulation is key to the pathogenesis of active Heymann nephritis. J Exp Med 1998;188(7):1353-8. DOI: 10.1084/jem.188.7.1353.
66 Seifert L, Hoxha E, Eichhoff AM, et al. The Most N-Terminal Region of THSD7A Is the Predominant Target for Autoimmunity in THSD7A-Associated Membranous Nephropathy. J Am Soc Nephrol 2018;29(5):1536-1548. DOI: 10.1681/ASN.2017070805;
67 Seifert L, Zahner G, Meyer-Schwesinger C, et al. The classical pathway triggers pathogenic complement activation in membranous nephropathy. Nat Commun 2023;14(1):473. DOI: 10.1038/s41467-023-36068-0.
68 Sethi S, Madden B. Mapping antigens of membranous nephropathy: almost there. Kidney Int 2023;103(3):469-472. DOI: 10.1016/j.kint.2023.01.003.
69 Siddique I, Di J, Williams CK, Markovic D, Vinters HV, Bitan G, Exophers are components of mammalian cell neurobiology in health and disease, bioRxiv 2021.12.06.471479; DOI: 10.1101/2021.12.06.471479;
70 Sidhom K, Obi PO, Saleem A. A Review of Exosomal Isolation Methods: Is Size Exclusion Chromatography the Best Option? Int J Mol Sci. 2020 Sep 4;21(18):6466. doi: 10.3390/ijms21186466.
71 Somogyi P, Takagi H. A note on the use of picric acid-paraformaldehyde-glutaraldehyde fixative for correlated light and electron microscopic immunocytochemistry. Neuroscience 1982;7(7):1779-83. DOI: 10.1016/0306-4522(82)90035-5.
72 Stanescu HC, Arcos-Burgos M, Medlar A, et al. Risk HLA-DQA1 and PLA(2)R1 alleles in idiopathic membranous nephropathy. N Engl J Med 2011;364(7):616-26. DOI: 10.1056/NEJMoa1009742;
73 Stricher F, Macri C, Ruff M, Muller S, HSPA8/HSC70 chaperone protein, Autophagy, 9:12, 2013, 1937-1954, DOI: 10.4161/auto.26448
74 Takemoto M, Asker N, Gerhardt H, et al. A new method for large scale isolation of kidney glomeruli from mice. Am J Pathol 2002;161(3):799-805. DOI: 10.1016/S0002-9440(10)64239-3.
75 Tinevez JY, Perry N, Schindelin J, et al. TrackMate: An open and extensible platform for single-particle tracking. Methods 2017;115:80-90. DOI: 10.1016/j.ymeth.2016.09.016.
76 Tomas NM, Beck LH, Jr., Meyer-Schwesinger C, et al. Thrombospondin type-1 domain-containing 7A in idiopathic membranous nephropathy. N Engl J Med 2014;371(24):2277-2287. DOI: 10.1056/NEJMoa1409354;
77 Tomas NM, Hoxha E, Reinicke AT, et al. Autoantibodies against thrombospondin type 1 domain-containing 7A induce membranous nephropathy. J Clin Invest 2016;126(7):2519-32. DOI: 10.1172/JCI85265;
78 Tomas NM, Meyer-Schwesinger C, von Spiegel H, et al. A Heterologous Model of Thrombospondin Type 1 Domain-Containing 7A-Associated Membranous Nephropathy. J Am Soc Nephrol 2017;28(11):3262-3277. DOI: 10.1681/ASN.2017010030.
79 Tomas NM, Huber TB, Hoxha E. Perspectives in membranous nephropathy. Cell Tissue Res 2021. DOI: 10.1007/s00441-021-03429-4;
80 Turek M, Banasiak K, Piechota M, et al. Muscle-derived exophers promote reproductive fitness. EMBO Rep 2021;22(8):e52071. DOI: 10.15252/embr.202052071;
81 Van der Walt S, Schonberger JL, Nunez-Iglesias J, et al. scikit-image: image processing in Python. PeerJ 2014;2:e453. DOI: 10.7717/peerj.453.
82 Van de Wakker SI, Meijers FM, Sluijter JPG and Vader P, Extracellular Vesicle Heterogeneity and Its Impact for Regenerative Medicine Applications, Pharmacological Reviews, 2023, 75(5) 1043-1061, DOI: 10.1124/pharmrev.123.000841;
83 Vaidya SR, Aeddula NR. Chronic Kidney Disease. [Updated 2022 Oct 24]. In: StatPearls [Internet]. Treasure Island (FL): StatPearls Publishing; 2024 Jan-. Available from: https://www.ncbi.nlm.nih.gov/books/NBK535404/;
84 Vallés-Saiz L, Peinado-Cahuchola R, Ávila J, Hernández F. Microtubule-associated protein tau in murine kidney: role in podocyte architecture. Cell Mol Life Sci. 2022 Jan 27;79(2):97, DOI: 10.1007/s00018-021-04106-z;
85 Wang Y, He YX, Diao TT, Wei SY, Qi WR, Wang CC, Song SM, Bi M, Li CM, Zhang CX, Hou YP, Wei QJ, Li B. Urine anti-PLA2R antibody is a novel biomarker of idiopathic membranous nephropathy. Oncotarget. 2017 Aug 3;9(1):67-74. doi: 10.18632/oncotarget.19859.
86 Wang Y, Arnold ML, Smart AJ, et al. Large vesicle extrusions from C. elegans neurons are consumed and stimulated by glial-like phagocytosis activity of the neighboring cell. Elife 2023;12. DOI: 10.7554/eLife.82227.
87 Wiech T, Reinhard L, Wulf S, et al. Bacterial infection possibly causing autoimmunity: Tropheryma whipplei and membranous nephropathy. Lancet 2022;400(10366):1882-1883. DOI: 10.1016/S0140-6736(22)02039-6.
88 Zhang, Y., Wu, KM., Yang, L. et al. Tauopathies: new perspectives and challenges. Mol Neurodegeneration 17, 28 (2022). https://doi.org/10.1186/s13024-022-00533-z;

## Claims

1. Method for the diagnosis, prognosis or monitoring, in vitro, of a disease involving proteostatic dysfunction, comprising the step of
a) detecting the presence or quantity of exophers in a cell or tissue sample or in a body fluid sample from a subject; and/or
b) analyzing the contents of exophers in a cell or tissue sample or in a body fluid sample.

2. The method according to claim 1, wherein the disease involving proteostatic dysfunction is a kidney disease, preferably chronic kidney disease, more preferably membranous nephropathy.

3. The method according to one of claims 1 or 2, wherein the exophers are kidney cell-derived exophers, preferably podocyte-derived exophers, and the sample is a urine sample.

4. The method according to one of the preceding claims, comprising a step of enriching the exophers in the respective body fluid sample.

5. The method according to one of the preceding claims, wherein the analysis of the contents of the exophers comprises the determination of a) the amount and characteristics of bound autoantibodies, b) the amount and characteristics of the protein contents, c) the amount and characteristics of the remaining molecules in the exopher, and/or d) the functionality of organelles and/or protein complexes.

6. The method according to one of the preceding claims, wherein step a) comprises the determination of the proportion of exophers in relation to the totality of extracellular vesicles in the sample, preferably a body fluid sample.

7. The method according to claim 1, wherein the sample is a cell or tissue sample, preferably a biopsy specimen, and wherein step a) comprises the imaging of the deposition pattern of exophers in the sample.

8. The method according to one of the preceding claims, wherein the subject is a human, preferably a human having, assumed to have or to develop a disease involving proteostatic dysfunction, preferably a kidney disease, more preferably a glomerular disease, more preferably chronic kidney disease, more preferably membranous nephropathy.

9. A method for the enrichment or isolation of human exophers, the method comprising the binding of the exophers present in a body fluid sample to anti-14-3-3, and/or anti-huIgG4 and/or anti-Hsc70 antibodies immobilized to a solid support.

10. An isolated human exopher.

11. An isolated human exopher according to claim 10, wherein the exopher is a kidney-cell-derived exopher, preferably a podocyte-derived exopher.
